(19) Europäisches Patentamt
European Patent Office
Office européen des brevets

(11) **EP 4 722 346 A1**

(12) **EUROPEAN PATENT APPLICATION**
published in accordance with Art. 153(4) EPC

(43) Date of publication:
08.04.2026 Bulletin 2026/15

(21) Application number: 24815589.7

(22) Date of filing: 30.05.2024

(51) International Patent Classification (IPC):
*C12N 5/077* (2010.01)    *A61K 35/34* (2015.01)
*A61P 9/04* (2006.01)     *C12N 5/0789* (2010.01)
*C12N 5/10* (2006.01)

(52) Cooperative Patent Classification (CPC):
A61K 35/34; A61P 9/04; C12N 5/06; C12N 5/10

(86) International application number:
PCT/JP2024/019949

(87) International publication number:
WO 2024/248109 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.05.2023 JP 2023088954

(71) Applicants:
• Heartseed Inc.
Tokyo 150-0023 (JP)

• Keio University
Tokyo, 108-8345 (JP)

(72) Inventors:
• TOHYAMA Shugo
Tokyo 160-8582 (JP)
• FUKUDA Keiichi
Tokyo 105-0023 (JP)

(74) Representative: Vossius & Partner
Patentanwälte Rechtsanwälte mbB
Siebertstrasse 3
81675 München (DE)

(54) **CARDIOMYOCYTE GROUP, PHARMACEUTICAL COMPOSITION, METHOD FOR PRODUCING CARDIOMYOCYTE GROUP, AND MYOCARDIAL SPHERE**

(57)    A cardiomyocyte population comprising cardiomyocytes differentiated from human pluripotent stem cells and having the following features (1), (2), and (3): (1) cells positive for cardiac troponin T constitute 90% or more of all viable cells,; (2) the spontaneous beating rate is 0 to 60 beats per minute; and (3) the population of cardiomyocyte is within 30 days from initiation of differentiation. Also provided is a pharmaceutical composition comprising the cardiomyocyte population and/or a cardiac spheroid obtained by spheroidizing the cardiomyocyte population, and a pharmaceutically acceptable carrier. Further provided is a method for producing the cardiomyocyte population, the method comprising: (a) expanding human pluripotent stem cells in culture; (b) culturing the expanded human pluripotent stem cells under conditions for differentiation into cardiomyocytes to produce a cell population comprising 60% or more cardiomyocytes; and (c) removing human pluripotent stem cells and non-cardiomyocyte cells from the cell population.

EP 4 722 346 A1

## Description

### FIELD OF INVENTION

The present invention relates to a population of cardiomyocytes, a pharmaceutical composition, a method for producing a population of cardiomyocytes, and a cardiac spheroid. This application claims the benefit of priority based on Japanese Patent Application No. 2023-088954, filed on May 30, 2023, the contents of which are hereby incorporated herein by reference in their entirety.

### BACKGROUND

When a large number of cardiomyocytes are lost due to a heart attack, the myocardial tissue eventually replaces with non-contractile fibrous tissue, leading to heart failure throughout the lifetime. Currently, the clinical application of cardiomyocytes derived from human pluripotent stem cells for cardiac repair is being investigated. It is expected that transplanting human pluripotent stem cell-derived cardiomyocytes (hereinafter referred to as "hPSC-CM") into myocardial tissue will regenerate cardiac muscle tissue. Regenerative medicine using hPSC-CM is considered promising because, in theory, large quantities of hPSC-CM can be produced in vitro.

To date, numerous studies, including those conducted by the inventors of the present invention, have disclosed methods for producing large quantities of high-purity human pluripotent stem cell-derived cardiomyocytes (see Patent Documents 1-3).Conceptual validation experiments involving the transplantation of these cardiomyocytes into animal models include, for example, experiments in which cardiomyocyte populations differentiated from human ES cells using activin A and BMP4 were transplanted into rats with induced myocardial infarction (see Non-Patent Literature 1), and experiments where cardiomyocytes differentiated from human iPS cells were transplanted into the infarct hearts of immunodeficient mice (e.g., see Non-Patent Literature 2). In these experiments, the survival of transplanted cardiomyocyte populations in the recipient heart over a certain period of time has been confirmed. Furthermore, to improve the engraftment rate of cardiomyocyte populations in recipient myocardial tissue, several strategies have been evaluated, including co-transplantation with Matrigel (registered trademark) and genetic modification of PSC-CM to promote cell proliferation. While these techniques have demonstrated efficacy in improving engraftment rates, most remain clinically unacceptable at present.

Furthermore, simply engrafting hPSC-CM into the recipient heart is insufficient for cardiac regeneration therapy. For cardiac regeneration therapy, electrical integration between transplanted cardiomyocytes and recipient cardiomyocytes is required in the damaged recipient heart.

Electrical integration between recipient cardiomyocytes and transplanted cardiomyocytes in the damaged recipient heart has been confirmed in experiments where human ES cell-derived cardiomyocytes were transplanted into the damaged hearts of guinea pigs (e.g., see Non-Patent Literature 3, 4, and Patent Documents 4). However, the findings obtained from these experiments cannot confirm whether clinical-scale cardiomyocyte transplantation can be performed safely and achieve myocardial regeneration, considering the clinical application of this technology, as the experimental animal models used were not physiologically appropriate animal models.

Experiments have also been conducted using model animals such as primates and pigs, which are relatively suitable for extrapolation to humans. However, cardiac myocytes differentiated from human pluripotent stem cells typically exhibit fetal or neonatal phenotypes. Therefore, significant differences in electrical characteristics have been reported between cardiac myocytes derived from human pluripotent stem cells and those from the human heart, particularly in terms of maturity. As a result, new issues have been reported, such as the limited survival rate of transplanted pluripotent stem cell-derived cardiomyocytes and the induction of transplant-related arrhythmias in the recipient's heart by pluripotent stem cell-derived transplanted cardiomyocytes (see, for example, Non-Patent Literature 5, 6, 7, and 8). To suppress transplant-related arrhythmias, methods such as reducing arrhythmias with antiarrhythmic drugs (see Non-Patent Literature 9) have been disclosed; however, these methods have issues such as being burdensome for the recipient. Additionally, cells with genes related to ion channels2 (see Non-Patent Literature 10) have been reported. However, using genetically edited cells as transplant cells poses risks such as unintended alterations at unintended sites due to gene editing, which could lead to undesirable mutations and unpredictable effects, as well as complex manufacturing and quality control processes, making them undesirable.

### PRIORART DOCUMENTS

### PATENT DOCUMENT

Patent Document 1: International Publication No. 2007/088874
Patent Document 2: International Publication No. 2016/010165
Patent Document 3: International Publication No. 2018/074457
Patent Document 4: U.S. Patent Application Publication No. 2020/0407687

Specification

## NON-PATENT LITERATURE

Non-Patent Literature 1: Laflamme MA. et al., Cardiomyocytes derived from human embryonic stem cells in pro-survival factors enhance function of infarcted rat hearts. (2007) Na Biotechnol., 25, 1015-1024.
Non-patent literature 2: Funakoshi S. et al., Enhanced engraftment, proliferation and therapeutic potential in heart using optimized human iPSC-derived cardiomyocytes. (2016) Sci Rep., 6, 19111.
Non-patent literature 3: Shiba Y., et al., Human ES-cell-derived cardiomyocytes electrically couple and suppress arrhythmias in injured hearts. (2012) Nature, 489, 322-5.
Non-patent literature 4: Dhahri W. et al., In Vitro Matured Human Pluripotent Stem Cell-Derived Cardiomyocytes Form Grafts With Enhanced Structure and Function in Injured Hearts. (2022) Circulation, 145, 1412-1426.
Non-patent literature 5: Liw YW. et al., Human embryonic stem cell-derived cardiomyocytes restore function in infarcted hearts of non-human primates. (2018) Nat Biotechnol., 36, 597-605.
Non-patent literature 6: Chong JJ. et al., Human embryonic-stem-cell-derived cardiomyocytes regenerate non-human primate hearts. (2014) Nature, 510, 2 73-7.
Non-patent literature 7: Shiba Y. et al., Allogeneic transplantation of iPS cell- derived cardiomyocytes regenerates primate hearts. (2016) Nature, 538, 388-391.
Non-patent literature 8: Romagnuolo R. et al., Human Embryonic Stem Cell-Derived Cardiomyocytes Regenerate the Infarcted Pig Heart but Induce Ventricular Tachyarrhythmias. (2019) Stem Cell Reports., 12, 967-981.
Non-patent literature 9: Nakamura K. et al., Pharmacologic therapy for engraftment arrhythmia induced by transplantation of human cardiomyocytes. (2021) Stem Cell Reports., 16, 2473-2487.
Non-patent literature 10: Marchiano S. et al., Gene editing to prevent ventricular arrhythmias associated with cardiomyocyte cell therapy. (2023) Cell Stem Cell., 30, 396-414.

## SUMMARY OF THE INVENTIONA PROBLEM TO BE SOLVED BY THE INVENTION

Human pluripotent stem cell-derived cardiomyocyte populations reported to date have not sufficiently satisfied all of the following conditions required for clinical application: (i) the ability to readily produce high-purity cardiomyocyte populations, (ii) the ability to safely transplant them into humans, (iii) the ability to electrically synchronize with recipient cardiomyocytes, maintain maturity, and survive long-term to regenerate damaged myocardial tissue, and(iv) a low incidence of severe ventricular arrhythmias persisting for a certain period of time in the recipient. Furthermore, the structural and functional characteristics required for human pluripotent stem cell-derived cardiomyocyte populations that meet the above conditions have not been elucidated through mechanisms of action or other means. Therefore, the only way to confirm whether such human pluripotent stem cell-derived cardiomyocyte populations meet the above conditions is to transplant them into myocardial tissue of primates or other species similar to humans.

Therefore, the present invention aims to provide human pluripotent stem cell-derived cardiomyocyte populations that can satisfy the conditions necessary for clinical application, cardiac spheroids formed from the cardiomyocyte populations, pharmaceutical compositions containing the cardiomyocyte populations and/or the cardiac spheroids as active ingredients, methods for manufacturing the cardiomyocyte populations, and methods for manufacturing the cardiomyocyte populations.

## MEANS FORSOLVING THE PROBLEM

The present invention includes the following aspects.

[1] A population of cardiomyocytes comprising ventricular cardiomyocytes differentiated from human pluripotent stem cells,
wherein the population of cardiomyocytes has the following characteristics:

(1) cells positive for cardiac troponin T constitute 90% or more of all viable cells,

(2) the spontaneous beating rate is 0 to 60 beats per minute, and

(3) the population of cardiomyocytes is a population within 30 days from initiation of differentiation.

[2] The population of cardiomyocytes according to [1], wherein the spontaneous beating rate is 0 to 50 beats per minute.

[3]A pharmaceutical composition comprising at least one selected from the group consisting of the population of cardiomyocytes according to [1] or [2], and cardiac spheroids obtained by spheroidizing the population of cardiomyocytes, and a pharmaceutically acceptable carrier.

[4] The pharmaceutical composition according to [3], wherein the proportion of viable cells in the population of cardiomyocytes is 80% or more of all cells.

[5] The pharmaceutical composition according to [3], wherein cells constituting the cardiac spheroids account for 60% or more of all cells contained in the pharmaceutical composition.

[6] The pharmaceutical composition according to [3], for use in the treatment of heart failure.

[7] A method for producing the population of cardiomyocytes according to [1]or [2], comprising:

(a) expanding human pluripotent stem cells in culture;

(b) culturing the expanded human pluripotent stem cells under conditions for differentiation into cardiomyocytes to produce a cell population comprising 60% or more cardiomyocytes; and

(c) removing human pluripotent stem cells and non-cardiomyocyte cells from the cell population.

[8] A cardiac spheroid obtained by spheroidizing the population of cardiomyocytes according to [1] or [2].

[9] The cardiac spheroid according to [8], wherein the diameter of the spheroid is 50 to 300 μm.

[10] A method for producing a cardiac spheroid, comprising suspending the population of cardiomyocytes according to [1] or [2] in a medium and subsequently statically culturing the suspension in a culture vessel having microwells at the bottom.

## ADVANTAGEOUS EFFECTS OF THE INVENTION

According to the present invention, there are provided a cardiomyocyte population derived from human pluripotent stem cells that can satisfy the conditions required for clinical application, a cardiac spheroid obtained by spheroidizing the cardiomyocyte population, a pharmaceutical composition comprising the cardiomyocyte population and/or the cardiac spheroid as an active ingredient, a method for producing the cardiomyocyte population, and a method for producing the cardiac spheroid.

## BRIEF DESCRIPTION OF THE DRAWINGS

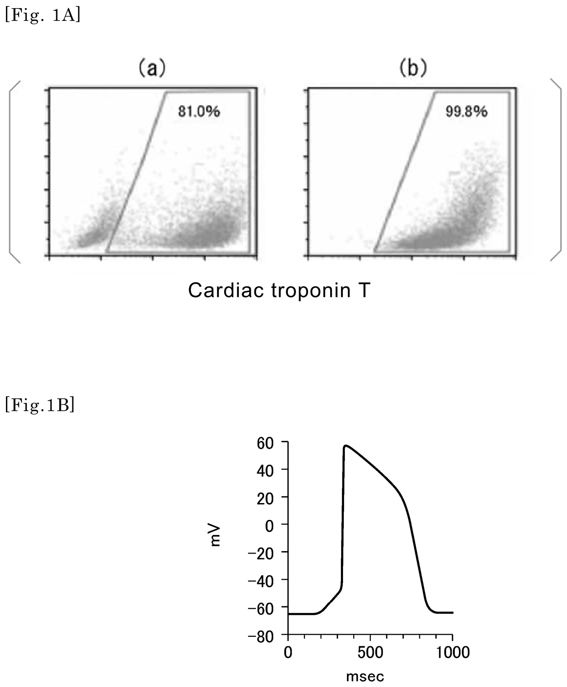

Fig. 1A(b) shows a result of flow cytometric analysis of the cardiac troponin T-positive rate of a human iPS cell-derived cardiomyocyte population prepared in Example 1(1). (a) shows a result of flow cytometric analysis of the cardiac troponin T-positive rate of the cell population before purification.

Fig.1B shows an action potential patterns obtained by patch clamp electrophysiological analysis of the human iPS cell-derived cardiomyocyte population prepared in Example 1(1).

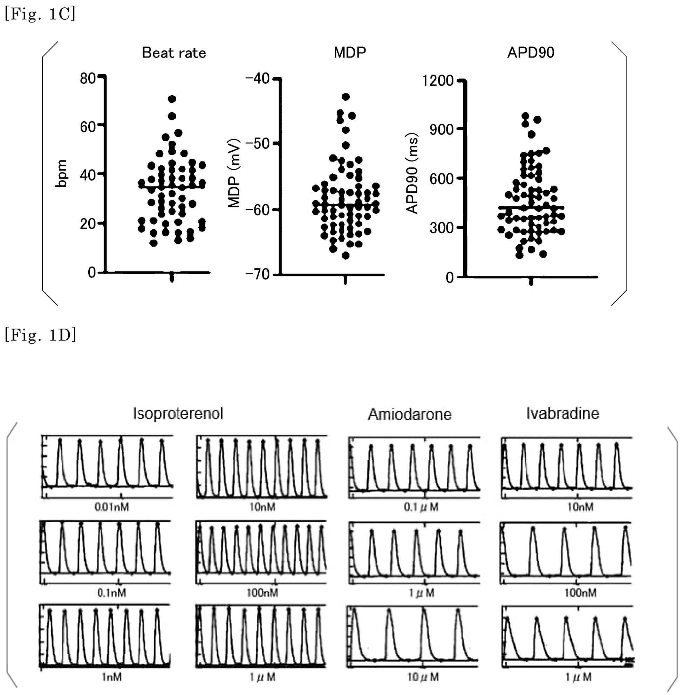

Fig.1C shows a spontaneous beat rate (Beat Rate), maximum diastolic potential (MDP), and action potential duration at 90% repolarization (APD90) obtained from electrophysiological analysis using the patch clamp method on the human iPS cell-derived cardiomyocyte population prepared in Example 1(1).

Fig.1D shows an action potential waveforms obtained from the analysis of drug responsiveness to isoproterenol, amiodarone, and ivabradine in the human iPS cell-derived cardiomyocyte population prepared in Example 1(1).

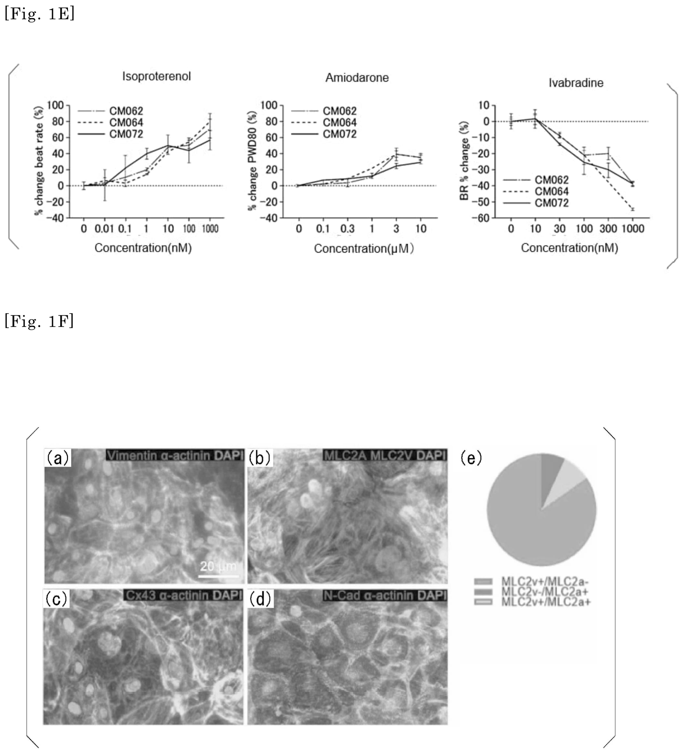

Fig. 1E shows a spontaneous beating rate per minute for the human iPS cell-derived cardiomyocyte population prepared in Example 1(1) in response to isoproterenol, amiodarone, and ivabradine.

Fig.1F shows a result of immunocytochemical analysis of the human iPS cell-derived cardiomyocyte population prepared in Example 1(1).

Fig.1F shows a result of immunocytochemical analysis of human iPS cell-derived cardiomyocytes prepared in Example 1(1).

Fig. 1G shows a result of measuring spontaneous beating rates in human iPS cell-derived cardiomyocytes (purified hiPSC-CMs) and unpurified cell populations (pre-purified hiPSC-CMs) produced in Example 1 (1).

Fig. 2A shows a survival rate of cardiomyocytes contained in cardiac spheroids produced in Example 2 (1).

Fig. 2B shows a result of measuring the sarcomere length of host cardiomyocytes and transplanted cardiomyocytes of *Cynomolgus monkeys* that were transplanted with cardiomyocytes produced in Example 2 (1) in Example 3.

Fig. 2C shows a result of long-term culture and immunohistochemical staining of cardiomyocytes produced in Example 2 (1) in Example 2 (2).Day 28: 28 days after the start of culture; Day 56: 56 days after the start of culture; Day 84: 84 days after the start of culture ; A, D, G: stained with CytoRed and DAPI; B, E, H:stained with cardiac troponin T (cTnT) and DAPI; C, F, I: stained with CytoRed, cTnT, and DAPI.

Fig. 2D shows a result of detecting bioluminescence signals in mature male NOG mice after transplantation of dispersed cardiomyocytes derived from human iPS cells expressing modified luciferase and modified luciferase-expressing cardiac spheroids (hiPSC-CSs) in Example 2(2).

Fig. 2E shows a graph of the bioluminescence signals detected in Figure 2D.

Fig. 2F shows a result of measuring the trough level of cyclosporine in the peripheral blood of a *Cynomolgus monkeys* transplanted with cardiac spheroids (equivalent to $2 \times 10^7$ cardiomyocytes) manufactured in Example 2 (1). The shaded area indicates the trough level of cyclosporine required to suppress acute rejection after cardiac transplantation.

Fig. 2G shows a result of measuring the trough levels of cyclosporine in the peripheral blood of a *Cynomolgus monkeys* transplanted with cardiac myoblasts (equivalent to $67 \times 10^7$ cardiac myocytes) produced in Example 2(1) of Example 3. The shaded area indicates the trough levels of cyclosporine used to suppress acute rejection in heart transplantation.

Fig. 3A shows a result of measuring the left ventricular shortening rate (FS) by cardiac echocardiography in *Cynomolgus monkeys* transplanted with cardiac myocytes or vehicles manufactured in Example 2(1), before transplantation, 4 weeks after transplantation (4w post-transplantation), and 12 weeks after transplantation (12w post-transplantation) in Example 3.

Fig. 3B shows a result of Holter electrocardiogram of a *Cynomolgus monkeys* transplanted with cardiac spheroids manufactured in Example 2(1), which exhibited transient ventricular tachycardia in Example 3.

Fig. 3C shows a result of Holter electrocardiogram of a *Cynomolgus monkeys* transplanted with cardiac spheroids or vehicles manufactured in Example 2(1) in Example 3.

Fig. 4A shows a histological examination results of *Cynomolgus* monkeys transplanted with cardiac spheroids manufactured in Example 2(1) in Example 3.

Fig. 4B shows a histological examination results of *Cynomolgus* monkeys transplanted with cardiac spheroids manufactured in Example 2(1) in Example 3.

Fig. 5A shows a result of echocardiograms of a *Cynomolgus* monkeys transplanted with cardiac spheroids or vehicles manufactured in Example 2(1) in Example 3, before transplantation, 4 weeks after transplantation (4w post-transplantation), and 12 weeks after transplantation (12w post-transplantation). The scale bar indicates 5 cm.

Fig. 5B shows a result of LVEF and FS measurements in *Cynomolgus* monkeys transplanted with cardiac spheroids (equivalent to $6 \times 10^7$ cardiomyocytes) or vehicles manufactured in Example 2(1) in Example 3, at pre-transplantation, 4 weeks post-transplantation (4w post-transplantation), and 12 weeks post-transplantation (12w post-transplantation).

Fig. 5C shows a result of Holter electrocardiogram of a *Cynomolgus monkeys* transplanted with cardiac spheroids or vehicles manufactured in Example 2(1) in Example 3.

Fig. 5D shows aresult of measuring the left ventricular shortening fraction (FS) by cardiac echocardiography in *Cynomolgus monkeys* transplanted with human iPS cell-derived cardiomyocytes or vehicles in Example 3. hiPSC-CSs (purified): cardiac spheroids manufactured in Example 2(1); hiPSC-CSs (unpurified): cardiac spheroids manufactured from hiPSC-CSs that have not been purified.

Fig. 5E shows a result of measuring cardiac troponin T (cTnT) and brain natriuretic peptide (BNP) in the serum of *Cynomolgus monkeys* transplanted with cardiac spheroids or vehicle from Example 2(1) in Example 3. A, B: Transplantation of $2 \times 10^7$ cardiomyocytes; C, D: Transplantation of $6 \times 10^7$ cardiomyocytes.

Fig. 5F shows a duration of arrhythmia in individual *Cynomolgus monkeys* that developed transient arrhythmia after transplantation of cardiomyocytes produced in Example 2(1) in *Example 3*. $*p < 0.05$ versus pre-Tx; $\#p < 0.05$ between vehicle and hiPSC-CSs.

Fig. 6 shows a histological examination results of the heart (A), lungs (B, F), liver (C, G), kidney (D, H), and spleen (E, I) in *Cynomolgus monkeys* transplanted with cardiac spheroids manufactured in Example 2(1) in Example 3. The scale bars in A-D indicate 1 cm. The scale bar in E indicates 5 mm. The scale bar in I indicates 200 $\mu$m.

## DETAILED DESCRIPTION OF THE PREFERRED EMBODIMENTS

Numerical ranges indicated by "~" mean ranges that include the numerical values described before and after "~" as lower and upper limits.

The term "comprise" means that the target components may include components other than the target components. The term "consist of" means that the target components do not include components other than the target components. The term "consist essentially of" (consist essentially of)" means that the components other than the target components are not included in a manner that performs a special function (such as a manner that completely loses the effect of the invention). In this specification, when "comprise" is used, it includes the "consist of" and "consist essentially of'.

The proteins, peptides, polynucleotides (DNA, RNA), vectors, cells, cell populations, and cardiomyocytes described herein may be isolated. "Isolated" means separated from their natural state or from other components. "Isolated" may mean that the component does not substantially contain other components. "Does not substantially contain other components" means that the amount of other components contained in the isolated component is negligible. The content of other components contained in isolated components may be, for example, 10 mass % or less, 5 mass % or less, 4 mass % or less, 3 mass % or less, 2 mass % or less, 1 mass % or less, 0.5 mass % or less, or 0.1 mass % or less. The proteins, peptides, polynucleotides (DNA, RNA), vectors, cells, cell populations, and cardiomyocytes described herein may each be isolated proteins, isolated peptides, isolated polynucleotides (isolated DNA, isolated RNA), isolated vectors, isolated cells, isolated cell populations, and isolated cardiomyocytes, respectively.

In this specification, the ratio (percentage) of specific cells in a cell population refers to the ratio of the number of specific cells to the total number of cells in the cell population, calculated as follows:

Specific cell ratio (%) = Number of specific cells / Total number of cells in the cell population $\times$ 100

**(Cardiomyocyte Population)**

The first embodiment of the present disclosure relates to a cardiomyocyte population comprising ventricular cardiomyocytes differentiated from human pluripotent stem cells. The cardiomyocyte population has the following characteristics (1), (2), and (3):

(1) cells positive for cardiac troponin T constitute 90% or more of all viable cells.
(2) The spontaneous beating rate is 0 to 60 beats per minute.
(3) The population of cardiomyocytes is within 30 days from the initiation of differentiation of differentiation.

The population of cardiomyocytes of the present embodiment, by having the above features (1), (2), and (3), constitutes a cardiomyocyte population capable of satisfying the following conditions (i) to (iv) required for clinical application:

(i) The cardiomyocyte population is highly pure and can be manufactured in a simple manner.
(ii) It can be safely transplanted into humans.
(iii) It can electrically synchronize with the recipient's cardiomyocytes, and survive long-term maintained maturity, thereby regenerating the damaged portion of the recipient's heart.
(iv) It has a low incidence of severe ventricular arrhythmias persisting for a certain period of time in the recipient.

The cardiomyocyte population of the present embodiment preferably satisfies one or more of the above conditions (i) to (iv), more preferably satisfies two or more, further preferably satisfies three or more, and particularly preferably satisfies all four. The cardiomyocyte population of the present embodiment can be considered a clinically applicable cardiomyocyte population because it satisfies the above conditions (i) to (iv).

**<Human Pluripotent Stem Cells>**

As used herein, "pluripotent stem cells" refer to cells having self-renewal capacity and multipotency. Specific examples of pluripotent stem cells include embryonic stem cells (ES cells), induced pluripotent stem cells (iPS cells), embryonic germ cells (EG cells), and germline stem cells (GS cells), as well as cells derived from these pluripotent stem cells that possess differentiated pluripotency. The term "pluripotent stem cells" is not specifically limited to cells with self-renewal capacity and multipotency, and may also include unknown cells having properties equivalent to those of the aforementioned ES cells or iPS cells.

Whether a cell is a pluripotent stem cell can be determined based on the presence or absence of properties specific to pluripotent stem cells or the expression of various markers specific to pluripotent stem cells. For example, properties specific to pluripotent stem cells include the ability to self-renew and the ability to differentiate into other cell types with properties different from those of pluripotent stem cells. Teratoma formation ability and chimera mouse formation ability are also examples of properties specific to pluripotent stem cells.

Various markers specific to pluripotent stem cells (hereinafter referred to as "pluripotent stem cell markers") are factors specifically expressed in pluripotent stem cells, such as Oct3/4, Nanog, Sox2, SSEA-1, SSEA-3, SSEA-4, TRA1-60, TRA1-81, Lin28, Fbx15, SSEA-5, GDF3, KLF4, and CLDN6. If the expression of at least one of these pluripotent stem cell markers is observed, the cell can be identified as a pluripotent stem cell. Pluripotent stem cell markers may be used alone or in combination of two or more. As one embodiment, cells expressing Oct3/4 may be identified as pluripotent stem cells. The expression of pluripotent stem cell markers in cells may be confirmed using known methods such as RT-PCR or microarray analysis.

Human pluripotent stem cells may be human ES cells, human iPS cells, human EG cells, or human GS cells. Among these, human ES cells or human iPS cells are preferred, and human iPS cells are more preferred. "Human iPS cells" are pluripotent stem cells artificially generated from non-pluripotent cells such as adult somatic cells, and methods for producing human iPS cells are well known in the art. Specifically, they can be produced by introducing one or more reprogramming factors into somatic cells such as fibroblasts, hematopoietic cells, or epidermal cells. Examples of reprogramming factors include Oct3/4, Sox2, c-Myc, 1-Myc, Klf4, Nanog, and Lin28. The pluripotency of the stem cells can be confirmed by detecting the expression of genes and/or proteins specific to the aforementioned stem cells, and such cells can be selected accordingly. Human iPS cells are provided in multiple cell lines by the Kyoto University iPS Cell Research Foundation (a public interest incorporated foundation located at 53 Shogoinkawaramachi, Sakyo-ku, Kyoto, Kyoto Prefecture, Japan). These human iPS cell lines may be used as human pluripotent stem cells. Specific examples of human iPS cell lines provided by the Kyoto University iPS Cell Research Foundation include the Ff-I01 strain, Ff-I14 strain, Ff-I01s01 strain, Ff-I14s03 strain, Ff-I14s04 strain, QHJI-I14s04 strain, Ff-MH09s01 strain, and Ff-MH15s02 strain. Among these, the Ff-I14 strain, Ff-I14s04 strain, and QHJI-I14s04 strain are preferred for use. The above iPS cells are all HLA haplotype homozygous human iPS cells. For clinical applications, it is preferable to use cell lines of clinical grade. Human iPS cells may also be obtained from cell banks such as the American Type Culture Collection (ATCC). Commercially available human iPS cells may also be used.

### <Cardiomyocyte Population>

The cardiomyocyte population of the present embodiment comprises cardiomyocytes (hPSC-CMs) differentiated from human pluripotent stem cells, and particularly includes mature ventricular cardiomyocytes. Cardiomyocytes are defined herein as cells positive for at least one of sarcomeric $\alpha$-actinin, cardiac troponin T (cTnT), or troponin I type 1 (TNNI1). Ventricular cardiomyocytes are defined as cells positive for cardiac troponin T and/or myosin light chain 2v (MLC2v). In addition, cardiomyocytes are typically cells exhibiting spontaneous beating activity. Ventricular cardiomyocytes are a type of cardiomyocytes that can constitute ventricular myocardium.

Furthermore, as used herein, "myocardial progenitor cells" refers to precursor cells of the cardiomyocytes, wherein at least one of Nkx2.5, GATA4, MEF2C, or MES1 is positive. In this specification, "non-myocardial cells" refers to cells that are neither cardiomyocytes nor myocardial progenitor cells, and specific examples include smooth muscle cells and endothelial cells.

The cardiomyocyte population of the present embodiment satisfies all of the following conditions:

(1) cardiac troponin T-positive cells constitute 90% or more of all viable cells;
(2) the spontaneous beating rate is 0 to 60 beats per minute; and
(3) the cardiomyocyte population is within 30 days or less from initiation of differentiation.

As used herein, "cell population" refers to a group of two or more cells of the same or different types. "Cardiomyocyte population" refers to a cell population containing cardiomyocytes, which may consist solely of cardiomyocytes or may further include non-cardiomyocyte cells. At least a portion of the cardiomyocytes contained in the cardiomyocyte population of the present embodiment are ventricular cardiomyocytes. The cardiomyocyte population may also include cardiomyocytes other than ventricular cardiomyocytes (e.g., cardiomyocyte progenitor cells, atrial cardiomyocytes, etc.). The production of the cardiomyocyte population of the present invention from human pluripotent stem cells may be carried out using the method described below.

### <(1) Cardiac troponin T-positive cells constitute 90% or more of all viable cells>

Cardiac troponin T (cTnT) is a protein that constitutes the myofibrils of cardiac muscle and is known as a cardiomyocyte marker. Cardiac troponin T-positive cells are considered to be cardiomyocytes. Human cardiac troponin T is a protein encoded by the TNN2 gene (Gene ID: 7139).

As used herein, "cells positive for" means that the target protein or gene is expressed in the cells at a detectable level using methods known in the art. Protein detection may be performed using immunological assays employing antibodies, such as ELISA, immunofluorescence staining, or flow cytometry. For proteins that are expressed within cells but not on the cell surface (e.g., transcription factors or subunits thereof), the target protein may be detected by co-expressing a reporter protein and detecting the reporter protein. Gene detection may be performed using nucleic acid amplification and detection methods, such as RT-PCR, microarray analysis, or RNAseq.

As used herein, "cells negative for" means that the expression level of the target protein or gene in the cell is below the detection limit of any of the methods described above. The detection limit may vary depending on the method used, but can be determined using conventional techniques.

The proportion of cardiac troponin T-positive cells in a cardiomyocyte population is not particularly limited as long as it is 90% or more of the total number of viable cells. Preferably, the proportion is 90% or more, more preferably 95% or more, further preferably 96% or more, even more preferably 97% or more, still more preferably 98% or more, and particularly preferably 99% or more or 99.5% or more of all viable cardiomyocytes. The percentage of troponin T-positive cells in the total number of viable cells may be, for example, 90-100%, 95-100%, 96-100%, 97-100%, 98-100%, 99-100%, or 99.5-100%.

The percentage of troponin T-positive cells in the total number of viable cells may be determined by measuring the number of troponin T-positive cells using any of the above methods and calculating the ratio with respect to the total number of viable cardiomyocytes, as determined by commonly used methods (e.g., gating in flow cytometry). The number of cardiac troponin T-positive cardiomyocytes is preferably confirmed by flow cytometry analysis using antibodies against cardiac troponin T. Antibodies against cardiac troponin T for flow cytometry are commercially available. For example, viable cardiomyocytes in a cardiomyocyte population may be selected using flow cytometry or similar methods, and the number of cardiac troponin T-positive cells among the selected viable cells may be measured to calculate the ratio of cardiac troponin T-positive cells to total viable cardiomyocytes. For example, in data obtained from flow cytometry analysis of a cardiomyocyte population, the viable cell population may be selected by gating, and the ratio of troponin T-positive cells may be obtained based on the expression level of troponin T in the selected viable cell population.

The selection of viable cells in the cardiomyocyte population may be carried out using methods such as forward scatter, side scatter, or staining with a vital dye (e.g., nucleic acid-binding dye, protein-binding dye).

### <(2) Spontaneous beating is 0 to 60 beats per minute>

The spontaneous beating of the cardiomyocyte population of the present embodiment is 0 to 60 beats per minute, preferably 0 to 50 beats per minute, more preferably 0 to 45 beats per minute, and particularly preferably 0 to 35 beats per minute. The spontaneous beating of the cardiomyocyte population may also be 10 to 60 beats per minute, 10 to 50 beats per minute, 10 to 45 beats per minute, 10 to 40 beats per minute, or 10 to 35 beats per minute. Furthermore, the spontaneous beating may be 20 to 60 beats per minute, 20 to 50 beats per minute, 20 to 45 beats per minute, 20 to 40 beats per minute, or 20 to 35 beats per minute.

The spontaneous beating rate of a cardiomyocyte population (i.e., the number of spontaneous beats per unit time) can be measured using methods known in the art. Measurement of the spontaneous beating rate may be performed using any method capable of analyzing cellular movement. Examples of such methods include obtaining images or videos of a cardiomyocyte population under a microscope and analyzing them using motion vectors, or recording potential patterns (spontaneous action potentials) that change in response to cellular beating. Specific examples include measuring the action potential duration at 90% repolarization (APD90) or spontaneous action potentials using a patch clamp amplifier, or using a multi-electrode system, calcium imaging, or impedance measurement.

The above spontaneous beating rate is preferably measured using an appropriate number of cells suitable for the above methods; multiple measurements may be performed, and the results may be statistically processed to obtain the values. Specifically, a method employing a patch clamp amplifier to record the action potential duration at 90% repolarization (APD90) and spontaneous action potentials of a cardiomyocyte population may be exemplified. The intracellular solution used to measure action potentials may include: potassium gluconate (130 mmol/L), KCl (10 mmol/L), NaCl (5 mmol/L), $MgCl_2$ (1 mmol/L), ethylene glycol-bis(β-aminoethyl ether)-N,N,N',N'-tetraacetic acid (EGTA: 0.1 mmol/L), magnesium-bound adenosine triphosphate (0.1 mmol/L), and 4-(2-hydroxyethyl)-1-piperazineethanesulfonic acid (HEPES: 10 mmol/L). The extracellular solution may include: NaCl (136.5 mmol/L), KCl (5.4 mmol/L), $CaCl_2$ (1.8 mmol/L), $MgCl_2$ (0.53 mmol/L), HEPES (85.5 mmol/L), and glucose (5.5 mmol/L).

### <(3) Within 30 days from initiation of differentiation>

As used herein, "initiation of differentiation" refers to the time point at which culture of human pluripotent stem cells is initiated in a differentiation induction medium for cardiomyocytes (i.e., a medium containing substance that induce differentiation into cardiomyocytes). "Within 30 days from initiation of differentiation" refers to the period from the initiation of such culture until 30 days have elapsed.

As described herein, the cardiomyocyte population of the present embodiment may be produced by: (A) a differentiation induction process from pluripotent stem cells to cardiomyocytes, and (B) a removal process of pluripotent stem cells and non-cardiomyocytes. The cardiomyocyte population of the present embodiment is one in which process (B) is completed within 30 days from the initiation of process (A). Conventionally, cells cultured for about 30 days from the initiation of differentiation induction of human pluripotent stem cells into cardiomyocytes correspond to the fetal-to-neonatal stage of human cardiomyocytes, and are smaller in size and less mature than adult cardiomyocytes. On the other hand, it is known that continuing culture for a longer period, such as 40 days or more, increases the maturity of cardiomyocytes (see, e.g., Dhahri W. et al., Circulation, 145:1412-1426 (2022)). However, long-term culture is costly and unsuitable for practical application, and problems such as increased non-cardiomyocytes and reduced cell adhesion during prolonged culture make it unsuitable for clinical use.

The cardiomyocyte population of the present embodiment possesses the characteristics described in (1) and (2) above even when the culture period is within 30 days from the initiation of differentiation induction into cardiomyocytes, and contains mature ventricular cardiomyocytes at high purity. Therefore, it is a cardiomyocyte population suitable for clinical application.

As further described below in the method for producing cardiomyocyte populations, a method in which the culture period from initiation of differentiation into cardiomyocytes to completion of purification is limited to 30 days or less to produce mature, high-purity cardiomyocytes is extremely advantageous for clinical use.

In the case where the cardiomyocyte population of the present embodiment is frozen and stored after being manufactured by processes (A) and (B) described above, the freezing and storage period is excluded from the period of "within 30 days from the initiation of differentiation." That is, the period from the start of freezing of the cardiomyocyte population to the completion of thawing of the frozen cardiomyocyte population is excluded from the period of "within 30 days from the initiation of differentiation."

**<Other features>**

Feature (2) of the cardiomyocyte population described above reflects the maturity of the cardiomyocytes, and the characteristic that "the spontaneous beating rate is 0 to 60 beats per minute" indicates that the population contains a large proportion of ventricular cardiomyocytes. In addition, since the presence of pacemaker cells and/or atrial cardiomyocytes within the population increases the beating rate, feature (2) also indicates that the content of such cells is low. The cardiomyocyte population of the present embodiment may further be characterized, instead of or in addition to feature (2), by containing a high proportion of cells positive for myosin light chain 2v (MLC2v), a marker for ventricular cardiomyocytes. Preferably, the proportion of MLC2v-positive cells is at least 70%, more preferably at least 80%, and most preferably at least 90% at the time the cardiomyocyte population of the present embodiment is transplanted into recipient myocardial tissue.

For clinical use, cardiomyocyte populations are typically cryopreserved and thawed prior to administration. Known cell cryopreservation solutions may be employed for this purpose, and such solutions are commercially available in various forms. Examples include STEM-CELLBANKER® GMP grade (manufactured by Zenogen Pharma Co., Ltd.). Cryopreservation may be performed by suspending the cardiomyocytes in a cryopreservation solution and subsequently freezing the suspension.

The cardiomyocyte populations of the present embodiment may also exhibit responsiveness to antiarrhythmic drugs. Examples of such drugs include adrenergic receptor agonists (e.g., isoproterenol), HCN channel blockers (e.g., ivabradine), and potassium channel blockers (e.g., amiodarone).

By having all of features (1) to (3), the cardiomyocyte population of the present embodiment can be clinically used as transplantable cardiomyocytes. The cardiomyocyte population having these features is a population of cardiomyocytes, particularly mature ventricular cardiomyocytes, of high purity. As demonstrated in the examples below, such cardiomyocytes can electrically synchronize with the recipient's native cardiomyocytes, maintain their maturity, survive long term, and regenerate damaged portions of the recipient's heart.

Furthermore, as shown in the examples below, transplantation of the cardiomyocytes of the present embodiment results in a cell population with a low incidence of prolonged and severe ventricular arrhythmias. Because these cardiomyocytes are derived from human pluripotent stem cells and are highly purified, they can be safely transplanted into humans. In addition, they can be manufactured in a simple and industrially advantageous manner. Accordingly, the cardiomyocytes of the present embodiment are highly useful as transplantable cardiomyocytes.

**(Cardiac Spheroid)**

The cardiomyocyte population of the present embodiment may also be used in the form of cardiac spheroids (hereinafter simply referred to as "cardiac spheroids"). The "cardiac spheroids" of the second embodiment of the present disclosure refer to aggregates of the above-described cardiomyocyte population, which may be formed by the methods described below.

The diameter of a cardiac spheroid is not particularly limited, provided that the cells near the center of the spheroid do not undergo cell death and do not become significantly less mature compared to peripheral cells. As an example, the diameter may be 50 μm to 300 μm. A preferred range is 100 μm to 250 μm, and a more preferred range is 150 μm to 200 μm. While spheroids are preferably spherical, any shape is acceptable as long as the aggregate maintains the viability of the constituent cardiomyocytes. The term "diameter of a cardiac spheroid" refers to the distance across the aggregate through its center point. For elliptical aggregates, the minimum diameter should be within the lower limit of the above range and the maximum diameter should be within the upper limit. In populations containing multiple cardiac spheroids, it is preferable that at least 60%, more preferably at least 70%, and most preferably at least 80% of the spheroids fall within the above-mentioned diameter range, though spheroids of other sizes may also be present.

The diameter and distribution of cardiac spheroids can be measured by known methods, such as image analysis of spheroid photographs or particle size distribution measurement devices. The spheroids may be aggregates composed solely of cardiomyocytes, or they may include cells aggregated around a microcarrier-like material suitable for transplantation (e.g., extracellular matrix particles).

The cardiac spheroids of the present embodiment may serve as the active ingredient of the pharmaceutical compositions described below. In such cases, it is preferable that the cells constituting the cardiac spheroids account for at least 60%, more preferably at least 70%, and most preferably at least 80% of all cells in the pharmaceutical composition. The number of cells within a cardiac spheroid can be measured by any suitable method. For example, a correlation between spheroid diameter and cell number may first be established, and the number of cells in a test sample may then be calculated based on spheroid diameters and counts. This correlation may be obtained, for example, by measuring the diameter of multiple spheroids, dissolving them to release individual cardiomyocytes, and counting the cells. The maximum spheroid diameter may be used for such measurements.

Cardiac spheroids of the present embodiment may be produced from cardiomyocyte populations according to the first embodiment. The spheroids themselves need not be within 30 days from initiation of differentiation, although the cardiomyocyte populations used to produce the spheroids may be prepared within 30 days of differentiation initiation.

The inventors have found that when cardiomyocytes are produced in spheroid form, cell survival is maintained at a high level. Accordingly, by forming cardiomyocyte populations into spheroids and recovering them, populations with a high ratio of viable cells can be obtained. Cardiac spheroids (spheroid populations) produced from cardiomyocyte populations of the first embodiment contain mature cardiomyocytes with high purity and viability. When transplanted into a recipient, they readily engraft into the recipient's myocardium and exhibit a low incidence of prolonged, severe ventricular arrhythmias. Thus, they are suitable for clinical application.

**(Pharmaceutical Composition)**

A third embodiment of the present disclosure is a pharmaceutical composition comprising at least one type selected from the group consisting of the cardiomyocyte population related to the first embodiment and the cardiac spheroids related to the second embodiment, and a pharmaceutically acceptable carrier. The pharmaceutical composition of the present embodiment contains at least one type selected from the group consisting of the population of cardiomyocytes and the cardiac spheroids formed by spheroidization of the cardiomyocytes as an active ingredient. The pharmaceutical composition of the present embodiment preferably has a viable cell ratio of 60% or more in all cells contained in the pharmaceutical composition, and more preferably 70% or more.

The viable cell ratio is preferably maintained throughout the storage period and/or transportation time when the pharmaceutical composition is stored or transported, and further preferably maintained until immediately before administration to the patient. The number of viable cells can be measured by known methods. For example, viable cells and dead cells can be distinguished by Trypan blue staining, and the number of viable cells and dead cells can be counted separately, and the viable cell ratio can be calculated from the ratio of the number of viable cells to the total number of cells.

Furthermore, the cardiomyocyte population contained in the pharmaceutical composition of the present embodiment is preferably such that the ratio of cells constituting cardiomyocytes to the total number of cells contained in the pharmaceutical composition is 60% or more, 70% or more is further preferred, and 80% or more is even more preferred. The ratio of cardiomyocytes constituting the cardiac spheroid is preferably maintained throughout the storage period and/or transportation time when the pharmaceutical composition is stored and/or transported, and is further preferably maintained until just before administration to the recipient.

**<Cardiomyocyte population and cardiac spheroid>**

Cardiomyocyte populations and cardiac spheroids are the cardiomyocyte population according to the first embodiment of the present disclosure and the cardiac spheroids according to the second embodiment of the present disclosure.

**<Pharmaceutically acceptable carrier>**

"Pharmaceutically acceptable carrier" means a carrier that does not inhibit the physiological activity of the active ingredient and does not exhibit substantial toxicity to the target organism. "Does not exhibit substantial toxicity" means that the component does not exhibit toxicity to the target organism at the usual dosage. In the pharmaceutical composition of the present embodiment, the pharmaceutically acceptable carrier is a carrier that does not damage the cardiomyocyte population according to the first embodiment and does not exhibit substantial toxicity to the target organism. The pharmaceutically acceptable carrier includes any known pharmaceutically acceptable component that is typically considered an inactive component. Pharmaceutically acceptable carriers are not particularly limited, but examples include solvents, diluents, vehicles, excipients, flow promoters, binders, granulating agents, dispersing agents, suspending agents, wetting agents, lubricants, disintegrants, solubilizers, stabilizers, emulsifiers, fillers, and the like. Pharmaceutically acceptable carriers may be used alone or in a combination of two or more.

Pharmaceutically acceptable carriers may be buffers. Examples of buffers include physiological saline solution, general buffers (e.g., phosphoric acid, citric acid, and other organic acids), etc.

The pharmaceutical composition may further contain other components in addition to the above components. Other components are not particularly limited and may be used without restriction in the pharmaceutical field. Examples of other components include pharmaceutical excipients other than those mentioned above. Pharmaceutical excipients include, for example, preservatives (e.g., antioxidants), chelating agents, flavor and odor modifiers, sweeteners, thickening agents, buffering agents, colorants, etc., but are not limited to these.

The dosage form of the pharmaceutical composition is not particularly limited and may be any dosage form commonly used in pharmaceutical formulations. The pharmaceutical composition of the present embodiment is typically a non-oral formulation and may be formulated in a dosage form suitable for the specific administration methods described below, such as an injection formulation or a myocardial sheet. The pharmaceutical composition of the present embodiment may be frozen for storage and transportation as desired. When the pharmaceutical composition of the present embodiment is frozen, it may be thawed prior to use and further diluted with a sterile carrier corresponding to the target cell type. The pharmaceutical composition of the present embodiment may be supplied for administration to a patient, for example, by being sealed in a vial, but may also be sealed in a syringe, etc., depending on the administration method. Additionally, after administration of the pharmaceutical composition of the present embodiment, an immunosuppressant that is typically used may be administered to the patient who received the administration. The immunosuppressant may be selected appropriately according to the patient, but examples include agents that act on immunophilin (e.g., cyclosporine, tacrolimus hydrate, etc.). The pharmaceutical composition of the present embodiment may also be formulated as a combination with these immunosuppressive agents.

The administration route of the pharmaceutical composition of the present embodiment is preferably non-oral administration. As a non-oral administration route, any method known per se for administering cells to a patient may be used, for example, any method that allows cells to be administered to the patient's heart in a minimally invasive manner. Specifically, local administration to the heart (particularly the ventricles or myocardial tissue) is preferred, such as administration via a transplant needle from the epicardial side to the ventricular myocardial tissue, or administration via a catheter from the endocardial side or pericardium.

The pharmaceutical composition can administer a therapeutically effective amount of the cardiomyocytes according to the first embodiment and/or the cardiac spheroids according to the second embodiment. "Therapeutically effective amount" means the amount of the drug that is effective for the treatment or prevention of the target disease. For example, the therapeutic effective amount of the cardiomyocytes and/or the cardiac spheroids may be an amount effective for cardiac repair in the recipient. The therapeutic effective dose may be appropriately determined based on the patient's symptoms, weight, age, gender, etc., as well as the formulation of the pharmaceutical composition and the administration method. For example, the pharmaceutical composition may be administered as a single dose of $10^5$ to $10^{10}$ cardiomyocytes, preferably $10^7$ to $10^9$ cardiomyocytes.

The pharmaceutical composition may be administered as a single dose or as multiple doses. For example, after administering the pharmaceutical composition to a patient, the patient's cardiac function and the engraftment of the transplanted cardiomyocytes may be monitored, and the pharmaceutical composition may be administered again as necessary.

The target for administration of the pharmaceutical composition of the present embodiment is humans. The pharmaceutical composition can be used for the treatment of heart diseases such as heart failure. Heart failure is a disease characterized by impaired cardiac function. Causes of heart failure include, for example, myocardial infarction, angina pectoris, hypertension, valvular heart disease, cardiomyopathy, arrhythmia, and congenital diseases. The heart failure targeted for treatment may be caused by any of these causes. The pharmaceutical composition of the present embodiment can be suitably applied for the treatment of heart failure caused by the aforementioned causes, where heart muscle tissue is damaged. By administering the pharmaceutical composition of the present embodiment (transplantation of cardiomyocytes and/or cardiac spheroids) to subjects suffering from heart failure, damaged heart muscle tissue can be regenerated, thereby improving cardiac function. The pharmaceutical composition of the present embodiment specifically has the following functions: (1) the transplanted contractile cardiomyocytes adhere to the damaged myocardium and directly enhance the contractile force of the damaged region of the recipient heart, and/or (2) the release of paracrine factors having functions such as angiogenesis, cardioprotective effects, anti-inflammatory effects, and anti-fibrotic effects.

**(Method for manufacturing a population of cardiomyocytes)**

A fourth embodiment of the present disclosure provides a method for manufacturing a population of cardiomyocytes. The method for manufacturing a population of cardiomyocytes according to this embodiment includes the following processes (a) to (c), in the order listed.

(a) a process of expanding human pluripotent stem cells in culture;
(b) a process of culturing the expanded human pluripotent stem cells under conditions for differentiation into cardiomyocytes to produce a cell population containing a sufficient proportion of cardiomyocytes, specifically a proportion of 60% or more of cTnT-positive cells, such that the proportion reaches 90% or more after process (c); and
(c) a process of removing human pluripotent stem cells and non-cardiomyocytes from the cell population.

**<Process (a)>**

The expanded culture of human pluripotent stem cells can be performed by known methods. Examples include the methods described in Thomson et al., Science (1998) 282(5391):1145-7; Hovatta et al., EE; Ludwig et al., Nat Methods (2006) 3:637-46; Kennedy et al., Blood (2007) 109:2679-87; Nat Methods (2011) 8:424-9; Wang et al., Stem Cell Res. (2013) 11(3):1103-16; and International Publication No. 2018/181342, among others.

The culture medium may be prepared using a medium commonly used as a basal medium for mammalian cell culture. Examples of basal media include BME medium, BGJb medium, CMRL 1066 medium, Glasgow MEM medium, Improved MEM Zinc Option medium, IMDM medium, Medium 199, Eagle MEM medium, αMEM medium, DMEM medium, F-12 medium, DMEM/F12 medium, IMDM/F12 medium, Ham medium, RPMI 1640 medium, and Fischer's medium, and mixtures of these media may also be used.

Alternatively, commercially available basal media for stem cell culture may be employed. Examples include StemFitAS103C medium (Ajinomoto Co., Inc.), StemFit® AK media (e.g., StemFit AK03N, StemFit AK02N) (Ajinomoto Co., Inc.), Essential 8 medium (Life Technologies Corporation), mTESR1 medium (STEMCELL Technologies Canada Inc.), TESR2 medium (STEMCELL Technologies Canada Inc.), RHBS medium (STEMCELL Technologies Canada Inc.), TeSR™-E6 (STEMCELL Technologies Canada Inc.), hESF-GRO medium (Nipro Corporation), hESF-DIF medium (Nipro Corporation), CSIT-7 (Cell Science Research Institute Co., Ltd.), Essential 6 medium (Life Technologies Corporation), and the like.

The culture medium is preferably a chemically defined medium (CDM) to avoid contamination with chemically undefined components, and serum-free media are even more preferable. "Serum-free medium" refers to a medium that does not contain unadjusted or unpurified serum. Media containing purified blood-derived components and/or purified animal tissue-derived components (e.g., growth factors such as bFGF) are also included within the definition of serum-free medium.

Serum-free medium may contain serum substitutes. Examples of serum substitutes include serum albumin, transferrin, fatty acids, collagen precursors, trace elements, 2-mercaptoethanol, or 3'-thioglycerol. Such substitutes may be commercially available products, such as Knockout™ Serum Replacement (Life Technologies Corporation), Chemically-defined Lipid Concentrate (Life Technologies Corporation), GlutaMAX™ (Life Technologies Corporation), B27 (Life Technologies Corporation), and N2 (Life Technologies Corporation), but are not limited thereto.

The culture medium may further optionally contain some or all of the essential amino acids other than L-tryptophan (e.g., L-leucine, L-lysine, L-phenylalanine, L-isoleucine, L-threonine, L-histidine, L-methionine, and L-valine),

The culture medium may further optionally contain some or all of the non-essential amino acids (e.g., L-alanine, L-arginine, L-asparagine, L-aspartic acid, glycine, L-glutamine, L-glutamic acid, L-cysteine, L-serine, L-tyrosine, and L-proline), and other amino acids such as L-cysteine.

The culture medium may further optionally contain natural amino acids such as L-cystine in addition to the above-mentioned twenty amino acids.

The culture medium may further contain additives. Examples include ROCK (Rho-associated protein kinase) inhibitors such as Y-27632, antibiotics such as penicillin-streptomycin, vitamins, L-ascorbic acid, magnesium L-ascorbate phosphate, sodium pyruvate, 2-aminoethanol, glucose, sodium bicarbonate, HEPES, insulin, progesterone, sodium selenite, putrescine, and the like. Additives are preferably included within their known concentration ranges.

The medium may contain fatty acids. Examples include oleic acid, linoleic acid, α-linolenic acid, γ-linolenic acid, palmitic acid, stearic acid, arachidonic acid, eicosapentaenoic acid, docosahexaenoic acid, butyric acid, acetic acid, valeric acid, caproic acid, enanthic acid (heptanoic acid), caprylic acid, pelargonic acid, capric acid, lauric acid, myristic acid, pentadecanoic acid, margaric acid, xanthic acid, eleostearic acid, 8, 11-eicosadienoic acid, 5,8,11-eicosatrienoic acid, behenic acid, lignoceric acid, nerotic acid, cerotic acid, montanic acid, mericinic acid, and the like. Fatty acids may be saturated or unsaturated.

The culture medium may also be a basal medium containing L-ascorbic acid, selenium, transferrin, insulin, FGF2, and TGFβ1, to which L-tryptophan or L-tryptophan derivatives are added.

The pH of the culture medium is preferably about 6.0 to about 8.5, more preferably about 7.0 to about 7.5. The culture medium is preferably sterilized by filtration using a membrane filter or the like.

Human pluripotent stem cells may be cultured using conventional methods, with or without feeder cells such as fibroblasts.

The culture vessels used are not particularly limited as long as they are suitable for cell culture. Examples include flasks, tissue culture flasks, dishes, Petri dishes, multi-dishes, microplates, microwell plates, multi-plates, multi-well plates, micro slides, chamber slides, tubes, trays, culture bags, and roller bottles.

Culture vessels may be cell-adhesive or non-cell-adhesive. Adhesive culture vessels may be coated with substrates such as Matrigel (Niwa A, et al. PLoS One. 6(7): e22261, 2011), gelatin, collagen, elastin, fibronectin, vitronectin, laminin, hyaluronic acid, chondroitin sulfate, glycosaminoglycans, proteoglycans, or artificial materials mimicking their functions. Culture is preferably performed by adherent culture using such coated containers.

The culture temperature is about 30-40 °C, preferably about 37 °C. The $CO_2$ concentration is about 1-10%, preferably about 2-5%. The oxygen concentration is generally 1-40%, but may be appropriately adjusted according to culture conditions.

**<Process (b)>**

Any method capable of obtaining the cardiomyocyte population of the present embodiment may be employed as a method for inducing differentiation of human pluripotent stem cells into cardiomyocytes. Examples include: (i) activating Wnt/β-catenin signaling in human pluripotent stem cells to obtain a first cell population (culture in a first differentiation medium), and (ii) inhibiting Wnt/β-catenin signaling in the first cell population to obtain a second cell population containing cardiomyocyte precursors (culture in a second differentiation medium). Thereafter, the cardiomyocyte precursor cells are further cultured for 1 to 5 days in media such as RPMI, DMEM, or StemPro-34 to obtain a population of cardiomyocytes including mature ventricular cardiomyocytes. Through these processes, a cell population in which 60% or more of all viable cells have differentiated into cardiomyocytes can be obtained.

Activation of Wnt/β-catenin signaling may be achieved by exposing pluripotent stem cells to Wnt agonists such as activin A, BMP4, or bFGF. Wnt agonists include GSK-3β inhibitors such as CHIR-99021, TW-119, SB216763, SB415286, and CHIR-98014. These may be employed at concentrations allowing cardiomyocytes with the desired characteristics to be obtained. Cells are cultured in the first differentiation medium for 1-6 days. Differentiation into mesodermal cells may be confirmed by expression of markers such as T, MIXL1, and NODAL.

The first differentiation medium is then replaced with the second differentiation medium containing Wnt antagonists and VEGF. Wnt antagonists include IWP-2, IWP-3, IWP-4, IWR-1, PNU-74654, XAV939, and KY02111, among others. These may be employed at concentrations allowing cardiomyocytes with the desired characteristics to be obtained. Cells are cultured in the second differentiation medium for 1-7 days. The total culture period for steps (i) and (ii), including the subsequent 1-5 days of culture in RPMI, DMEM, or StemPro-34 and the culture period of process (c), is preferably within 30 days.

The culture containers and conditions used may be the same as those described for process (a). Differentiation is preferably performed by the method of Tohyama S. et al., Stem Cell Reports (2017) 9:1406-1414. Suitable containers include four-layer plates (Thermo Fisher Scientific), and suitable media include StemFit AS301 or StemFit AS501 (Ajinomoto Co., Inc.).

The proportion of cardiomyocytes in the cell population obtained by this process is not particularly limited, as long as it is sufficient to result in a cTnT-positive cell ratio of 90% or higher after process (c). Specifically, when purifying for about 4 days in process (c), cardiomyocytes preferably account for 60% or more at the end of process (b). When process (c) is performed to further purify cardiomyocytes, a lower ratio may also be employed. The proportion of cardiomyocytes may be determined by measuring the ratio of cells positive for markers such as cardiac troponin T by flow cytometry.

**<Process (c)>**

The method for removing human pluripotent stem cells and non-cardiomyocytes from the cell population after process (b) can be performed using known methods. For example, see International Publication No. 2018/3031074457, Tanosaki S. et al., iScience. 2020;23:101535, Tanosaki S. et al., STAR Protocols. 2022;3:101360, Tohyama S. et al., Cell Metabolism. 2016;23:663-74, and Tohyama S. et al., Cell Stem Cell. 2013;12:127-37, etc.

Specifically, a method for inducing cell death of residual undifferentiated cells and non-cardiomyocytes is exemplified by culturing the cell population obtained in process (b) in a medium containing at least one compound selected from the group consisting of fatty acid synthesis inhibitors, fatty acid utilization inhibitors, and cholesterol synthesis inhibitors.

As fatty acid synthesis inhibitors, those that inhibit fatty acid synthesis by targeting at least one factor selected from the group consisting of ATP citrate lyase, fatty acid synthase, acetyl-CoA carboxylase, and malonyl-CoA decarboxylase are preferred, and those that inhibit fatty acid synthesis by targeting at least one factor selected from the group consisting of ATP citrate lyase and fatty acid synthesis enzymes are preferred. As fatty acid synthesis inhibitors that inhibit fatty acid synthesis by targeting fatty acid synthase, orlistat (Orlistat), C75, flavonoids, and epigallocatechin-3-gallate (EGCG) are examples of fatty acid synthesis inhibitors that target fatty acid synthesis enzymes to inhibit fatty acid synthesis, with orlistat and C75 being preferred, and orlistat being more preferred.

As fatty acid synthesis inhibitors targeting ATP citrate lyase, LY294002 and SB204990 are examples.

As fatty acid synthesis inhibitors targeting acetyl-CoA carboxylase to inhibit fatty acid synthesis, sorafen A (Soraphen A), TOFA, A769662, metformin (Metformin), and AICAR are examples, with TOFA and A769662 being preferred.

As fatty acid utilization inhibitors, those that inhibit fatty acid utilization by targeting carnitine palmitoyl transferase 1 are preferred.

As fatty acid degradation inhibitors that target carnitine palmitoyl transferase 1 and inhibit fatty acid degradation, etomoxir (Etomoxir), perhexiline (Perhexiline), and ranolazine (Ranolazine) are examples, with etomoxir and perhexiline being preferred.

As cholesterol synthesis inhibitors, compounds that inhibit cholesterol synthesis by targeting at least one factor selected from the group consisting of acetyl-CoA acetyltransferase, HMG-CoA synthase, and HMG-CoA reductase, and cholesterol synthesis inhibitors that inhibit cholesterol synthesis by targeting at least one factor selected from the group consisting of acetyl-CoA acetyltransferase, HMG-CoA synthase, and HMG-CoA reductase are preferred, and cholesterol synthesis inhibitors that inhibit cholesterol synthesis by targeting HMG-CoA reductase are more preferred. As cholesterol synthesis inhibitors that inhibit cholesterol synthesis by targeting HMG-CoA reductase, pravastatin (Pravastatin), simvastatin (Simvastatin), fluvastatin (Fluvastatin), atorvastatin (Atorvastatin), pitavastatin (Pitavastatin), rosuvastatin (Rosuvastatin), cerivastatin (Cerivastatin), lovastatin (Lovastatin), and mevastatin (Mevastatin) are examples, with pravastatin, simvastatin, fluvastatin, atorvastatin, pitavastatin, and rosuvastatin being preferred, and simvastatin being more preferred.

Among these, it is preferable to use one or more selected from the group consisting of orlistat, C75, LY294002, SB204990, etomoxir, perhexiline, simvastatin, and salts thereof, and it is more preferable to use orlistat.

The concentrations of fatty acid synthesis inhibitors, fatty acid utilization inhibitors, and cholesterol synthesis inhibitors in the culture medium may be appropriately selected depending on their type。 For example, the concentration of the aforementioned fatty acid synthesis inhibitors or fatty acid utilization inhibitors may be 0.1 to 500 $\mu$M. The concentration of cholesterol synthesis inhibitors may be 0.01 to 50 $\mu$M.

For example, the medium used in the present invention may be prepared by adding at least one substance selected from the group consisting of fatty acid synthesis inhibitors, fatty acid utilization inhibitors, and cholesterol synthesis inhibitors, and at least one compound selected from the group consisting of cholesterol synthesis inhibitors, to the aforementioned basic culture medium to prepare the culture medium used in this process. The culture medium may optionally contain at least one compound selected from the group consisting of glucose, glutamine, and methionine.

The inclusion of glucose, glutamine, and/or methionine is expected to promote good cell growth.

The cell culture in process (c) can be performed under conditions generally used for cell culture. The culture temperature is approximately 30 to 40°C, preferably approximately 37°C. The $CO_2$ concentration is approximately 1 to 10%, preferably approximately 2 to 5%. Oxygen concentration is typically 1-40%, but may be selected appropriately depending on culture conditions, etc.

The culture time is not particularly limited, but is preferably 24 hours or longer, more preferably 48 hours or longer, with specific examples including 1-5 days.

As needed, the cells may be passaged. By culturing the cell population obtained in process (b) in a medium containing at least one compound selected from the group consisting of fatty acid synthesis inhibitors, fatty acid utilization inhibitors, and cholesterol synthesis inhibitors, human pluripotent stem cells and non-cardiomyocytes can be removed from the cell population obtained in process (b).

Furthermore, by adding the following process: culturing the cells using a medium that does not contain glucose, glutamine, or glutamic acid in the composition of a medium typically used for cell culture, and contains lactate, it is possible to selectively proliferate cardiomyocytes and induce cell death in non-cardiomyocytes. Culturing in such a medium further purifies the cardiomyocytes. This method is specifically described in International Publication No. 2007/088874 and International Publication No. 2016/010165.

Note that the feature "within 30 days after the initiation of differentiation" in the first embodiment of the cardiomyocyte population refers to the period from the start of the differentiation induction medium (medium containing substances that induce differentiation into cardiomyocytes; e.g., the first differentiation medium) as the starting date, and the period from the start of the culture in the medium containing the substance that induces differentiation of cardiomyocytes to the end of process (c) is within 30 days.

**<Optional process>**

**(Selection process)**

The manufacturing method of the present embodiment may optionally include a selection process (selection process) for selecting a population of cardiomyocytes having the characteristics described in (1), (2), and (3) above, after the above processes (a) to (c).

The selection process may be performed using the method described in the section on "population of cardiomyocytes" above. Specifically, for example, the following may be performed. Select a cell group from the cell group obtained in process (c) that has initiated differentiation (started process (b)) within 30 days (d1).

Next, select a population of cardiomyocytes in which cardiac troponin T-positive cells account for 90% or more of all viable cells. For example, perform flow cytometry analysis of the cell group obtained in process (c) using an antibody against cardiac troponin T, and select the cell group in which the ratio of troponin T-positive cells among all viable cells is 90% or more (d2).

Next, select a population of cardiomyocytes with spontaneous beats of 0-60 beats per minute from the previously selected cell group. For example, using a patch clamp amplifier, record the action potential duration (APD90) at 90% repolarization and spontaneous action potentials of the cardiomyocyte population, and select the cell population with a spontaneous beating rate of 0 to 60 beats per minute (d3). In this manner, the selection process can be performed. The processes (d1) to (d3) are not limited to the order described above and may be performed in any order.

**(Freezing process of cardiomyocyte population)**

The manufacturing method of the present embodiment may include a process of freezing the cardiomyocytes after the above processes (a) to (c) and after the optional selection process. The cardiomyocytes obtained by the above method are preferably suspended in a cell cryopreservation solution commonly used for cell cryopreservation after removing the final culture medium, detaching them from the culture plate by a known method, and washing them as necessary. Examples of cell cryopreservation solutions include STEM-CELLBANKER® GMP grade (manufactured by Zenogen Pharma Co., Ltd.) and others.

Here, the concentration of cardiomyocytes in the frozen suspension of cells to be cryopreserved should be such that cell death does not occur due to freezing and thawing; specifically, the viability of viable cells after freezing and thawing should be 50% or more of the viability of viable cells before freezing. The concentration of cardiomyocyte populations in the cell suspension is, for example, $1 \times 10^5$ to $1 \times 10^8$ cells/mL, preferably $1 \times 10^6$ to $1 \times 10^7$ cells/mL. For freezing containers, vials or bags used for freezing cells, etc., can be used.

Freezing and cryopreservation may be performed using conventional methods. For example, the cardiomyocytes may be frozen at -60°C to -80°C in a freezer, then transferred to a container filled with liquid nitrogen or to a deep freezer at -150°C or lower for long-term storage. Additionally, using a programmable freezer to freeze cells while adjusting the temperature is also preferable. For example, cooling at a rate of 1-10°C/min from -40°C to -60°C, holding at -40°C to -60°C for 10-60 minutes, then cooling at a rate of 1-10°C/min to -80°C, and maintaining at -80°C for several days are methods that can be employed. The thawing process for frozen cardiomyocytes can be performed using known methods. For example, frozen cells can be removed from a liquid nitrogen storage container or deep freezer and thawed in a 37°C water bath.

**(Method for manufacturing cardiac spheroids)**

The fifth embodiment of the present disclosure is a method for manufacturing cardiac spheroids. The method of the fifth embodiment includes a static culture process of a population of cardiomyocytes according to the first embodiment in a culture medium in a culture vessel having a bottom surface with a microwell. Cardiac spheroids according to the second embodiment can be manufactured by the manufacturing method according to the fifth embodiment.

The formation of cardiac spheroids can be performed by suspending the cardiomyocytes according to the first embodiment in a culture medium and statically culturing them in a culture vessel having a bottom surface with microwells. Note that the cardiomyocytes according to the first embodiment used here are not in the form of spheroids. The cardiomyocytes according to the first embodiment may be those produced by the manufacturing method according to the fourth embodiment.

As the culture medium, the aforementioned basal medium may be used. The culture medium preferably uses serum-free medium. The culture medium contains insulin (0.1-10 mg/L), transferrin (0.1-10 μg/L), selenium (0.1-10 μg/L), basic fibroblast growth factor (bFGF; 1 ng/mL to 100 ng/mL), epidermal growth factor (EGF; 1 ng/mL to 1000 ng/mL), platelet-derived growth factor (PDGF; 1 ng/mL to 1000 ng/mL), and endothelin-1 (ET-1) ($1 \times 10^{-8}$ to $1 \times 10^{-6}$ M). Specific examples of culture media include StemFit AS301 medium (manufactured by Ajinomoto Co., Inc.).

As a culture vessel with micro-wells on the bottom surface, commercially available culture vessels for spheroid formation can be used. Microwells refer to wells with an opening diameter in the micrometer range (e.g., 100-1,000 μm, preferably 100-800 μm, more preferably 200-500 μm, more preferably 300-500 μm). The culture container is preferably one with a low-adhesion culture surface. The culture container may also be coated with a bioadhesion inhibitor such as Prevlex® (manufactured by Nissan Chemical Industries, Ltd.). Specific examples of culture containers include a 6-well plate coated with Prevlex (Elplasia RB 500 400 NA 6, manufactured by Corning).

The manufacture of cardiac spheroids may be performed according to the prior art (e.g., International Publication No. WO2009/017524; Kawaguchi S. et al., JACC Basic Transl Sci. 2021;6:239-254; Tabei R. et al., J Heart Lung Transplant. 2019;38:203-214).

**(Method for treating heart failure)**

The sixth embodiment of the present disclosure is a method for treating heart failure, comprising administering at least one type selected from a group comprising a population of cardiomyocytes according to the first embodiment and a group of cardiac spheroids according to the second embodiment to a subject requiring treatment for heart failure.

The cardiomyocyte population and/or cardiac spheroids administered may be in the form of a pharmaceutical composition according to the third embodiment.

The cardiomyocyte population and/or cardiac spheroids may be administered to the subject by, for example, directly injecting them into myocardial tissue by injection. The administration device may be any device capable of injecting the cardiomyocyte population and/or cardiac spheroids into the target tissue, such as those described in International Publication No. WO2020/013125. A needle with a gauge of 24 to 30 is used. Catheters, etc., may also be used.

The cardiomyocytes and/or cardiac spheroids can be administered to patients requiring treatment for heart failure at a "therapeutically effective dose." The therapeutically effective dose may be determined appropriately based on the extent of myocardial tissue damage in the patient. For example, the single dose of cardiomyocyte populations and/or cardiac spheroids may be $10^5$ to $10^{10}$ cardiomyocytes, with $10^7$ to $10^9$ being preferred. Here, when administering cardiac spheroids, the cell dose is counted as the number of cardiomyocytes in the cardiomyocyte populations that form the cardiac spheroids.

The treatment method of the present embodiment is applicable to humans. Heart failure is a disease characterized by impaired cardiac function. Causes of heart failure include, for example, myocardial infarction, angina pectoris, hypertension, valvular heart disease, cardiomyopathy, arrhythmia, and congenital disorders. The heart failure to be treated may be caused by any of these causes. The pharmaceutical composition of the present embodiment can be suitably applied for the treatment of heart failure caused by the aforementioned causes, in which myocardial tissue is damaged. By administering the pharmaceutical composition of the present embodiment (transplantation of cardiomyocytes and/or cardiac spheroids) to a subject suffering from heart failure, damaged myocardial tissue can be regenerated, thereby improving cardiac function. The pharmaceutical composition of the present embodiment specifically has (1) a function to enhance the contractile force of the damaged site of the recipient heart by transplanted contractile cardiomyocytes adhering to the damaged myocardium, and/or (2) a function to release paracrine factors having angiogenic, cardioprotective, anti-inflammatory, and anti-fibrotic effects, etc.

**<Other Embodiments>**

Other embodiments of the present disclosure include the use of at least one type selected from the group consisting of the population of cardiomyocytes according to the first embodiment and the cardiac spheroids according to the second embodiment in the manufacture of a pharmaceutical composition for the treatment of heart failure. Other embodiments of the present disclosure include at least one type selected from a group consisting of the population of cardiomyocytes according to the first embodiment and the cardiac spheroids according to the second embodiment, for use in treating heart failure.

Other embodiments of the present disclosure include the use of at least one type selected from a group consisting of the population of cardiomyocytes according to the first embodiment and the cardiac spheroids according to the second embodiment, in the treatment of heart failure.

**EXAMPLES**

The present invention will now be described with reference to the following examples. However, the present invention is not limited to these examples.

**Example 1: Preparation and Analysis of Human iPS Cell-Derived Cardiomyocyte Population**

**(1) Preparation of human iPSC-derived purified cardiomyocyte population**

Human iPS cells (Ffl14s04 strain) (provided by the Kyoto University iPS Cell Research Foundation) were cultured in a culture medium containing iMatrix 511 (Nippi Inc.) and differentiated into cardiomyocytes using a differentiation induction protocol. The human iPS cells (Ffl14s04 strain) were maintained in StemFit AS103C medium (Ajinomoto Co., Inc.) on iMatrix 511 (Nippi Inc.)-coated culture plates. Differentiation induction into cardiomyocytes was performed on 4-layer culture plates (Thermo Fisher Scientific Inc.) using StemFit AS301 medium (Ajinomoto Co., Inc.) according to the method described in Tohyama S. et al., Stem Cell Reports 2017;9:1406-1414. Following differentiation induction, residual human iPS cells in the cell population obtained after differentiation were removed using orlistat, a fatty acid synthesis inhibitor, according to the methods described in Tanosaki S. et al., iScience 2020;23:101535, and Tanosaki S. et al., STAR Protoc 2022;3:101360. Subsequently, using StemFit AS501 (Ajinomoto Co., Inc.), the cells were cultured in accordance with the methods described in Tohyama S. et al., Cell Metab 2016;23:663-74, and Tohyama S. et al., Cell Stem Cell 2013;12:127-37. The purified population of cardiomyocytes was cryopreserved using STEM-CELLBANKER® GMP grade (Zenogen Pharma Inc.). Differentiation induction culture was carried out for 7-15 days. Culture for removal of residual human iPS cells was carried out for 5-10 days.

**(2) Flow cytometry and electrophysiological analysis of human iPSC-derived cardiomyocyte population**

The human iPS cell-derived cardiomyocyte population prepared and cryopreserved in (1) was thawed according to the methods described in Tohyama S. et al., Cell Metab 2016;23:663-74, and Tohyama S. et al., Cell Stem Cell 2013;12:127-37. The results are shown in Figures 1A-1F.

Figure 1A(b) shows the results of flow cytometric analysis of the cardiac troponin T (cTnT) positivity rate in the cardiomyocyte population after thawing. Dead cells were excluded by gating, and the cTnT positivity rate in viable cells was determined. The cardiomyocyte population prepared in (1) had a cTnT positivity rate of 99.8% after thawing (Figure 1A(b)). Note that the cTnT positivity rate of the cardiomyocyte population before purification was 81.0% (Figure 1A(a)).

Additionally, the thawed cardiomyocyte population was analyzed electrophysiologically using the patch-clamp technique according to the method described by Ichimura H. et al., Sci Rep 2020;10:11883. Specifically, the frozen cardiomyocyte population prepared in (1) was thawed, replated, and cultured for an additional 4 days. To examine the autonomous beating rate, the action potential duration at 90% repolarization (APD90) and spontaneous action potentials were recorded using a ruptured whole-cell patch-clamp technique with a patch-clamp amplifier (Axopatch 200B, Molecular Devices Inc.).

Action potentials were recorded in an intracellular solution containing potassium gluconate (130 mmol/L), KCl (10 mmol/L), NaCl (5 mmol/L), $MgCl_2$ (1 mmol/L), ethylene glycol -tetra acetic acid (EGTA: 0.1 mmol/L), magnesium-bound adenosine triphosphate (0.1 mmol/L), and 4-(2-hydroxyethyl)-1-piperazineethanesulfonic acid: HEPES (10 mmol/L). The extracellular bath solution contained NaCl (136.5 mmol/L), KCl (5.4 mmol/L), $CaCl_2$ (1.8 mmol/L), $MgCl_2$ (0.53 mmol/L), HEPES (85.5 mmol/L), and glucose (5.5 mmol/L).

The results of 65 analyses (65 cells) are shown in Figures 1B and 1C. As shown in Figure 1B, the cells of the human iPS cell-derived cardiomyocyte population prepared in (1) exhibited ventricular-like action potential patterns after freezing and thawing. As shown in the left panel (Beat Rate) of Figure 1C, the spontaneous beating rate was $34 \pm 13$ beats/min. As shown in the middle panel (MDP) of Figure 1C, the maximum diastolic potential (MDP) was $-58.4 \pm 0.64$ mV. As shown in the right panel (APD90) of Figure 1C, the action potential duration at 90% repolarization (APD90) was $465 \pm 25$ ms.

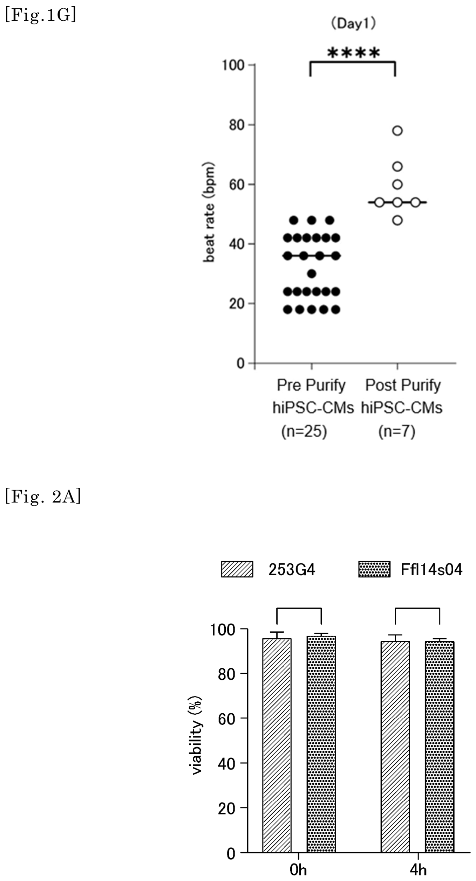

By contrast, in the cell population that underwent only the cardiomyocyte differentiation process without purification (n = 7), the results are shown in Figure 1G. As clearly indicated in Figure 1G, the average spontaneous beating rate per minute was approximately 38 beats/min in the purified cardiomyocyte population, whereas it was approximately 60 beats/min in the unpurified population.

**(3) Drug responses of human iPS cell-derived cardiomyocyte populations**

The human iPS cell-derived cardiomyocyte population prepared in (1) and cryopreserved was thawed and cultured in MEMα (Thermo Fisher Scientific, Inc., cat. no. 40610) containing 2.5% FBS (Merck Inc.) and sodium pyruvate (Merck Inc., cat. no. S8636) for approximately 1 week. On the day of assay, the cells were incubated in 5 mM Cal520AA (AAT Bioquest, cat. 21131) in HBSS (Thermo Fisher, cat. 1402504092) supplemented with pluronic F-127 (AAT Bioquest, cat. no. 20053) and probenecid (AAT Bioquest Inc., cat. no. 20062) at 37°C for 1 hour.

The culture medium was supplemented with the adrenergic receptor agonist isoproterenol (Merck Inc., cat. no. I6504) at concentrations of 0.01 nM, 0.1 nM, 1 nM, 10 nM, 100 nM, or 1 μM, and calcium transients were measured using FDSS/μCELL (Hamamatsu Photonics Inc.). Additionally, the culture medium was supplemented with the HCN channel blocker ivabradine (Merck Inc., cat. no. SML02C81) at concentrations of 10 nM, 30 nM, 100 nM, 300 nM, or 1 μM, and

calcium transients were measured in the same manner. Furthermore, the potassium channel blocker amiodarone (Merck Inc., cat. no. A8423) was added to the culture medium at concentrations of 0.1 $\mu$M, 0.3 $\mu$M, 1 $\mu$M, 3 $\mu$M, or 10 $\mu$M, and calcium transients were measured in the same manner.

The results are shown in Figures 1D and 1E. The analysis was performed on three different lots of cell populations prepared using the method described in (1). As clearly shown in Figures 1D and 1E, the human iPS cell-derived cardiomyocyte population obtained in (1) exhibited concentration-dependent responses to the adrenergic receptor agonist isoproterenol, the HCN channel blocker ivabradine, and the potassium channel blocker amiodarone.

### (4) Immunocytochemical analysis of human iPS cell-derived cardiomyocyte population

The human iPS cell-derived cardiomyocyte population prepared in (1) and cryopreserved was thawed according to the methods described in Tohyama S. et al., Cell Metab 2016;23:663-74, and Tohyama S. et al., Cell Stem Cell 2013;12:127-37. The thawed cardiomyocyte population was immunolabeled using $\alpha$-actin antibody, $\alpha$-vimentin antibody, and DAPI according to standard procedures. The results are shown in Figure 1F(a). Most of the cells in the cardiomyocyte population prepared in (1) were actin-positive and vimentin-negative.

Additionally, the same cell population was immunolabeled using anti-MLC2v antibody, anti-MLC2a antibody, and DAPI, and the results are shown in Figures 1F(b) and 1F(e). Of the cardiomyocyte population prepared in (1), 85% were mature ventricular cardiomyocytes positive for MLC2v alone, while 7% were immature cardiomyocytes positive for MLC2a alone or cells positive for both MLC2v and MLC2a (Figures 1F(b) and 1F(e)). Additionally, analysis of connexin 43 and N-cadherin expression revealed abundant expression of both (Figures 1F(c) and 1F(d)). Taken together, these findings demonstrate that the cardiomyocyte population prepared in (1) consists of nearly pure ventricular cardiomyocytes with a maturity level similar to that of adult cardiomyocytes.

### Example 2: Production and Analysis of Cardiac Spheroids

### (1) Production of cardiac Spheroids from human iPS cell-derived cardiomyocyte population

The human iPS cell-derived cardiomyocyte population cryopreserved in Example 1(1) was suspended in StemFit AS301 medium and cultured according to the methods described by Kawaguchi S. et al., JACC Basic Transl Sci 2021;6:239-254, or Tabei R. et al., J Heart Lung Transplant 2019;38:203-214, and seeded onto Prevelex® (Nissan Chemical Corporation)-coated 6-well plates (Elplasia RB500 400 NA 6, Corning Inc.), followed by 2 days of culture. After a total of 7 days of culture with half-medium changes every 2 days, human iPS cell-derived cardiac spheroids were harvested.

The size of the cardiac spheroids was measured using a particle size distribution analyzer (Beckman Coulter, Inc.). The results showed that the diameter of the cardiac spheroids was approximately 150 $\mu$m on average.

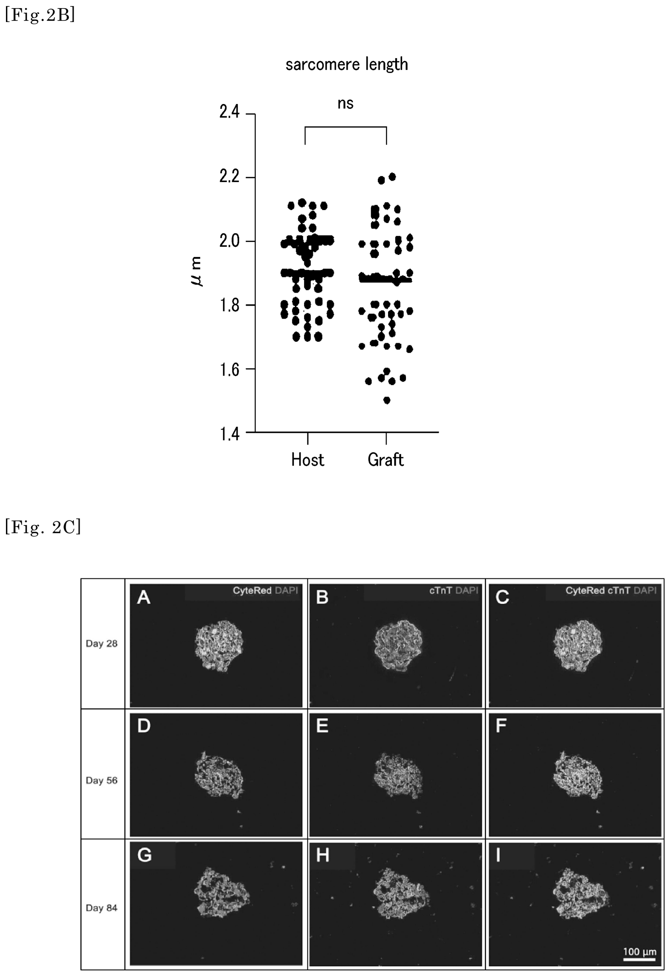

Additionally, portions of the cardiac spheroids were harvested immediately after collection and again 4 hours after collection. These spheroids were treated with a 3:1 solution of trypsin/EDTA and Accumax at 37°C for 10-15 minutes to dissociate the spheroids into single cells, followed by trypan blue staining. The survival rate of cardiomyocytes in individual spheroids is shown in Figure 2A. In Figure 2A, "253G4" represents the results obtained by preparing cardiomyocytes from a research-grade human iPS cell line (253G4, provided by Kyoto University) that was differentiated, purified, and frozen using the method described in Example 1(1), and then processed according to the method described in this example to form cardiac spheroids. As shown in Figure 2A, the survival rates of cardiomyocytes in spheroids stained with trypan blue immediately after collection (0 h) and 4 hours later (4 h) were over 90% for both the 253G4 strain and the Ffl14s04 strain. For the Ffl14s04 strain, the survival rate was 95.8 $\pm$ 2.2% (N = 3).

Furthermore, the number of cardiac spheroids with a diameter of 50 $\mu$m or larger in a fixed field of view of the recovered spheroid suspension was counted to calculate the number of cardiomyocytes constituting the spheroids. The number of cardiac spheroids with a diameter of 50 $\mu$m or smaller and the number of single cells (non-spheroid cardiomyocytes) in the same field of view were also counted to calculate the total number of cells. The ratio of cardiomyocyte numbers to total cell numbers was calculated using the following formula, and ranged from 74% to 86%.

Percentage of cardiomyocytes (%) = (Number of cardiomyocytes) / [(Number of cardiomyocytes) + (Number of non-cardiomyocytes)] $\times$ 100

### (2) Long-term culture of human iPS cell-derived cardiac spheroids

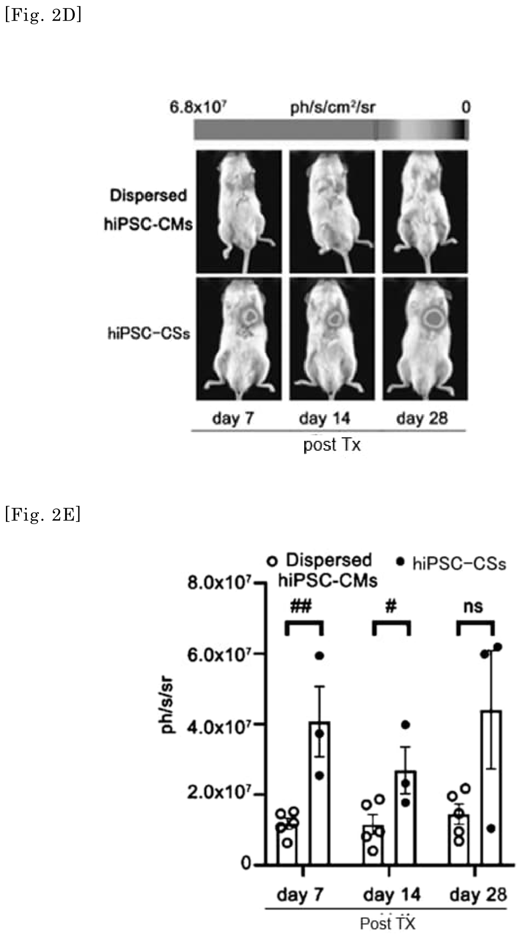

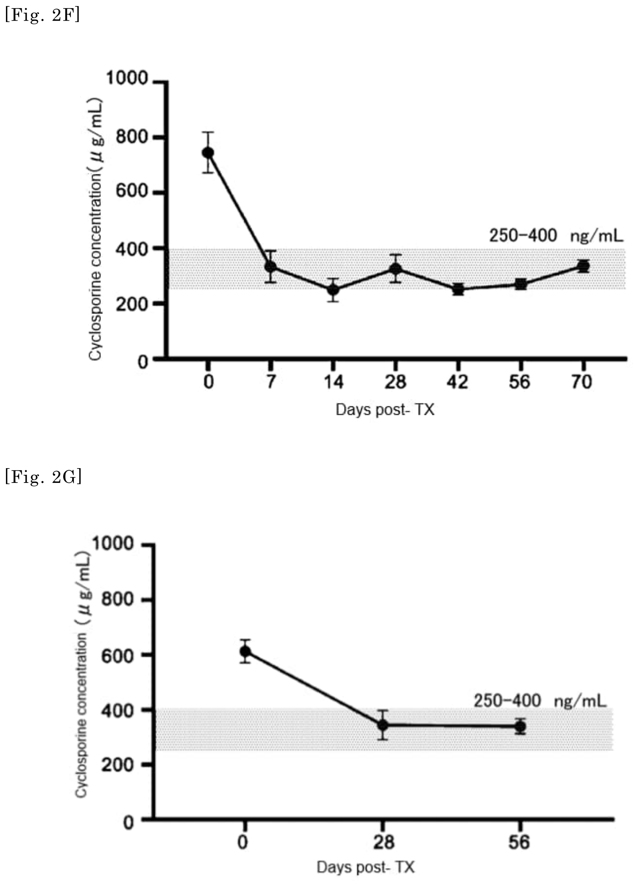

The cardiac spheroids obtained in (1) were further cultured in the same medium for an extended period. After 28 days, 56 days, or 84 days of culture, the cardiac spheroids were cultured for 1 hour using Cytored solution (molecular weight = 313.31, Fujifilm Wako Pure Chemical Co.). Additionally, the cardiac spheroids were embedded in Tissue-Tek O.C.T. Compound (SAKURA Inc.) and sectioned into 10 $\mu$m thick frozen sections using a cryostat (CM3050S, manufactured by Leica Microsystems Inc.). The sections were stained with $\alpha$-DAPI antibody, $\alpha$-human-specific cardiac troponin I antibody(cTnI, ab52862; Abcam Inc.) as primary antibodies, and biotin-conjugated secondary antibodies (Vector Laboratories Inc.) for immunohistological analysis. The results are shown in Figure 2C. As shown in Figure 2C, A, D, and G, it was observed that external culture medium, including CytoRed solution, penetrated through the central part of the cardiac spheroids, indicating that cardiomyocytes within the spheroids could directly receive nutrients from the external culture medium. Additionally, as shown in Figure 2C B, E, and H, the cardiac spheroids maintained the purity of cardiomyocytes even after 12 weeks of culture.

## (3) Transplantation of human iPS cell-derived cardiomyocytes into mature male NOG mice

Mature male NOG mice (In Vivo Science Co., Ltd.) were anesthetized with a low dose of isoflurane and ventilated using a rodent ventilator. A left thoracotomy was performed at the fourth intercostal space, and cardiac spheroids expressing modified luciferase (Akaluc) or $1\times10^6$ hiPSC-CSs expressing modified luciferase (Akaluc) were injected into the myocardium along with 60 $\mu$L of PBS. In these transplanted mice, anesthesia was administered 7-, 14-, and 28-days post-transplantation, and Akalumine n-Hydrochloride (Fujifilm Co., 20 nmol/g body weight) was administered intraperitoneally. Ten minutes after administration, imaging was performed using an imaging system (NEWTON 7.0 FT500, Vilber Inc.) to capture images at 20 cm $\times$ 20 cm with an exposure time of 1 minute, and image analysis was performed using Kuant software. The results are shown in Figures 2D and 2E. As clearly shown in Figures 2D and 2E, it was confirmed that cardiac spheroids transplanted into the animal's myocardium survived more effectively than cardiomyocytes transplanted into the animal's myocardium.

## Example 3: Cardiac Spheroids Transplantation into Cynomolgus Monkeys

### (1) Induction of myocardial ischemia-reperfusion injury in cynomolgus monkeys

Based on Japanese national regulations and guidelines, all experimental procedures were reviewed by the Committee for Animal Experiments and ultimately approved by the President of Shinshu University (No. 300023), Keio University (No. A2022-180) and Ina Research (No. 18088). Two weeks prior to cardiac spheroids transplantation, myocardial infarction was induced in a total of 10 cynomolgus monkeys (*Cynomolgus monkeys)* (4 males and 6 females) using the following method. The surgery was performed according to the methods described in Ichimura H. et al., Sci Rep. 2020;10:11883, or Kobayashi H. et al., Methods Mol Biol. 2021;2320:295-3023. Specifically, the cynomolgus monkeys were anesthetized by intra-muscular injections of ketamine and xylazine, intubated with a tracheal tube (diameter 3.5 mm), and ventilated with 2% isoflurane. Buprenorphine was routinely administered subcutaneously to reduce post-operative pain. During surgery, blood pressure, oxygen saturation, and electrocardiogram (ECG) were monitored. Phenylephrine was provided intravenously to maintain appropriate blood pressure. After median sternotomy, a 4-0 silk suture was passed through the myocardium at the mid-left anterior descending (LAD) coronary artery and threaded through a polyethylene tube. A silicone tube was placed on top of the polyethylene tube and tied off with sutures. Before induction of ischemia, 1 mg/kg of lidocaine and 200 U/kg of heparin were administered intravenously. Subsequently, the same dose of heparin was administered every hour until reperfusion. After 180 minutes of mid-LAD occlusion, the heart was repercussed by removing the tube.

### (2) Preparation and transportation of cardiac spheroids for transplantation

Cardiac spheroids prepared according to Example 2 (1) were collected on the day of transplantation, diluted with physiological saline, packaged in Eppendorf tubes, and transported to the transplantation facility at 4°C. Upon arrival at the transplantation facility, they were immediately transplanted into cynomolgus monkeys using the method described below.

### (3) Cardiac spheroids transplantation

Four male and six female cynomolgus monkeys, which had undergone myocardial infarction as described above, were divided into two groups with an equal male-to-female ratio. One group received the cardiac spheroids (equivalent to $2 \times 10^7$ cardiomyocytes) prepared in (2) above, while the other group received the vehicle using the following method. Euthanasia was performed at 12 weeks post-transplantation, and histological analysis was conducted. Cardiac function and arrhythmias were monitored during the observation period. Additionally, in a separate experiment, 10 cynomolgus monkeys (4 males and 6 females) induced with myocardial infarction were divided into two groups. One group received the cardiac spheroids (equivalent to $6 \times 10^7$ cardiomyocytes) described above (2), while the other group received the vehicle.

The specific transplantation method is as follows. On day 14 after the induction of myocardial infarction (MI), the animal underwent a second sternotomy and the heart was exposed. The cardiac spheroids from (2) suspended in physiological saline, or physiological saline vehicle, were delivered intra-myocardium into the infarct and border zones via 5-6 injections of 100 µL each using a 27-gauge injection needle. Immune suppression was achieved via intravenous injection of methylprednisolone and abatacept (Bristol Myers Squibb Co.) and subcutaneous injection of cyclosporine (Novartis International AG). A daily dosage of 50 mg/kg methylprednisolone was administered for 3 days starting on day -1 relative to transplantation. Subsequently, 2 mg/kg methylprednisolone was administered daily. Abatacept (Bristol Myers Squibb Co.) was administered at a dose of 12.5 mg/kg starting one day before transplantation and every two weeks thereafter. The daily dose of cyclosporine (Novartis International AG) was 5-10 mg/kg, and the dose was adjusted based on peripheral blood trough levels from day 5 post-transplantation until the end of the experiment. The cyclosporine tracer levels are shown in Figure 2F (cardiomyocytes equivalent to $2 \times 10^7$ cardiomyocytes transplanted) and Figure 2G (cardiomyocytes equivalent to $6 \times 10^7$ cardiomyocytes transplanted).

**(4) Analytical methods and procedures for animals after cardiac spheroids transplantation**

**(Confirmation of myocardial infarction model)**

Myocardial infarction was confirmed by transient ST elevation on the electrocardiogram and elevated serum cTnT levels.

**(Echocardiography)**

Echocardiography was performed on days 0, 28, and 84 after transplantation using a VIVID 7 system (GE Healthcare Biosciences Inc.). After intra-muscular injection of ketamine and xylazine, the heart was observed in short-axis view from the parasternal space. Left ventricular end-diastolic dimension (LVEDD) and left ventricular end-systolic dimension (LVESD) were measured in M-mode, and fractional shortening (FS) was calculated using the following equation: FS = $100 \times$ [(LVEDD - LVESD)/LVEDD].

**(Computed Tomography)**

Computed tomography (CT) was performed days -2, 28 and 84 relatives to transplantation. The CT imaging method was performed according to the method described by Shiba Y. et al., Nature. 2016; 538:388-391. Anaesthetized animals were intubated and mechanically ventilated with 2% isoflurane. The heart was imaged with R_mCT AX (Rigaku Corporation, Tokyo, Japan) while injecting a radiocontrast agent (Iopamidol) at 8 ml/min. Motion cycles of cardiac contraction and ventilation were automatically synchronized. Left ventricular end-diastolic volume (LVEDV) and end-systolic volume (LVESV) were measured using Ziostation2 software (Amin, Tokyo, Japan). Left ventricular ejection fraction (LVEF) was calculated using the following formula: LVEF = $100 \times$ [(LVEDV-LVESV)/LVEDV].

**(Holter Electrocardiography)**

The Holter electrocardiography recordings were performed on days - 4, 4, 7, 14, 28, 42, 56, 70 and 83 relatives to transplantation. The electrodes were placed in a 2-lead precordial system and connected to a Holter recorder. Animals were jacketed to protect the electrocardiography system and a 24-h electrocardiography was recorded. Ventricular tachycardia was defined as four or more consecutive premature ventricular complexes with a ventricular rate faster than 150 bpm. Sustained ventricular tachycardia was defined as ventricular tachycardia sustained longer than 30 seconds. All analyzes were performed by an operator who was blinded to the study groups.

**(Blood tests)**

Peripheral blood was collected and plasma was isolated to measure Brain natriuretic peptide (BNP) levels. Cardiac troponin T (cTnT) levels in the peripheral blood were measured on days - 12 (48 h after MI), and - 10 (96 h after MI). Whole blood was used to measure trough levels of cyclosporine by electrochemiluminescence immunoassay.

**(Histology)**

On day 84 post-transplantation, animals that received CSs were euthanized, and received full necropsy. The heart was harvested, transversely sliced to a 5-mm-thickness, and fixed with 4% paraformaldehyde. All sections were routinely stained with hematoxylin and eosin (HE) and picrosirius red (PSR) to determine the scar region. Scar area was calculated by subtracting graft area from all fibrous areas (shown in red by PSR staining) if the grafts were located in the scar. The sections were immunohistologically analyzed using primary antibodies against human-specific cardiac troponin I (cTnI, ab52862; Abcam Inc.), and biotin-conjugated secondary antibodies (Vector Laboratories, Inc.). For chromogenic detection, an HRP-conjugated streptavidin ABC kit (Vector Laboratories Inc.) was used, followed by a DAB substrate kit (Vector Laboratories Inc.).

The sections were immunohistologically analyzed using primary antibodies against myosin light chain 2A (MLK-2A; S 58-205,Becton, Dickinson and Company), myosin light chain 2V primary antibody (MLC-2V; ab92721, Abcam), anti-$\alpha$-actinin primary antibody(ab137346, Abcam), anti-vimentin primary antibody (Merck & Co.), anti-connexin 43 primary antibody (Cx43, ab11370; Abcam Inc.),anti-N-cadherin primary antibody (Thermo Fisher Scientific Inc.), anti-cTnT primary antibody (clone: 13-11), anti-CD45 primary antibody (clone: 2B11 & PD), anti-Ki67 primary antibody (ab16667, Abcam), Anti-pan-cadherin primary antibody (clone: CH-19), Anti-lycopersic lectin primary antibody(Tomato Lectin, Dylight 594 Labeled Tomato Lectin; Vector Laboratories, Inc.), and species-specific fluorescent dyes (Thermo Fisher Scientific, Inc.) were used for immunohistological analysis.

Brain, lung, liver, kidney, and spleen tissues were also collected and fixed in 4% PFA. Unless gross abnormalities were detected, several sections were randomly selected, and immunohistochemical staining was performed using HE and human-specific cTnI antibodies to detect the engraftment of ectopic cardiomyocytes. The stained sections were imaged using a NanoZoomer 2.0-RS (Hamamatsu Photonics Co., Ltd.) or a BZ-X700 microscope (KEYEN CE Co., Ltd.).

**(Statistical analysis)**

Echocardiography, CT, BNP, graft area and outcomes were analyzed by an analysis of variance (ANOVA), followed by post-hoc comparisons between time points by Tukey's multiple comparison test, and unpaired t-test analysis to compare groups at each time point. The percentage of cTnT was analyzed by ANOVA followed by post-hoc Tukey's multiple comparison tests. All summarized data are displayed as mean $\pm$ standard error of the mean. All statistical analyses were performed using GraphPad Prism (GraphPad Software Inc.), with the threshold for significance set at $P < 0.05$.

**(5) Analysis of animals after cardiac spheroids transplantation**

**(Histology)**

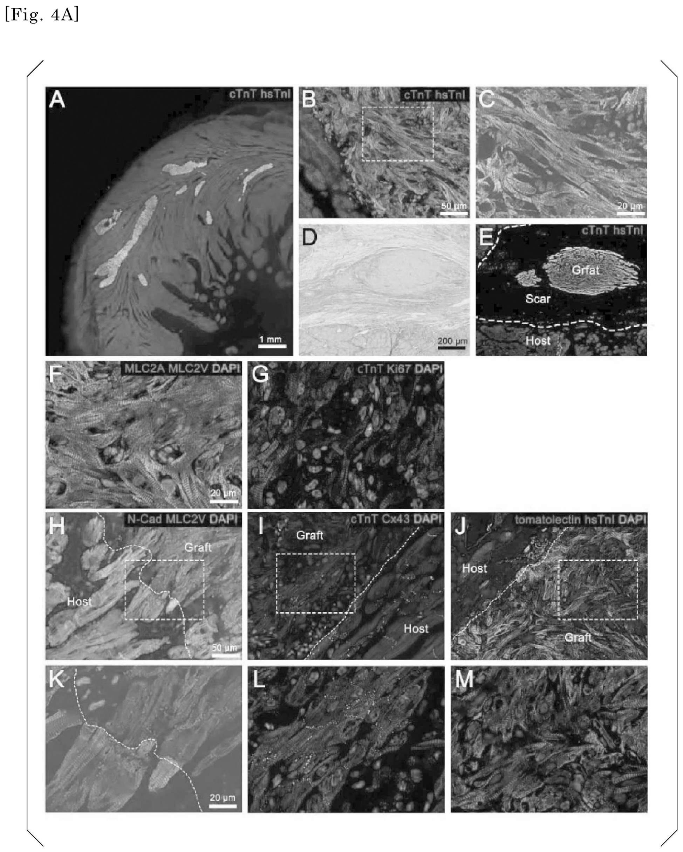

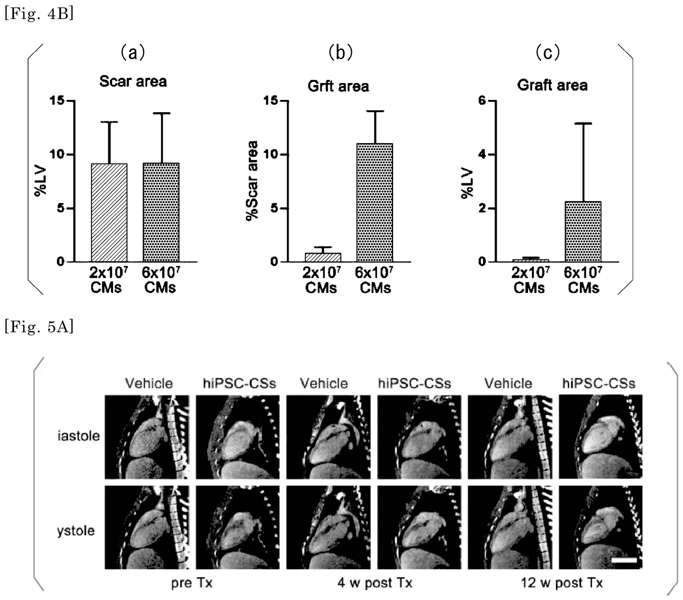

Hearts were collected from animals that received transplantation of cardiac spheroids (equivalent to $2 \times 10^7$ cardiomyocytes) on day 84 (12 weeks) post-transplantation, as described in Example 3(3), and stained with Picrosirius Red. The results showed that scar areas were not significantly different between the vehicle-transplanted animals and the spheroid of cardiomyocytes-transplanted animals (9.12 $\pm$ 1.74% vs. 5.24 $\pm$ 0.49% of the total left ventricular area, p = 0.15). When comparing animals transplanted with $6 \times 10^7$ cardiomyocytes to those transplanted with $2 \times 10^7$ cardiomyocytes, there was no difference in scar area between the two groups (Figure 4B(a)).The transplanted area was 13 times larger in animals transplanted with $6 \times 10^7$ cardiomyocytes compared to those transplanted with $2 \times 10^7$ cardiomyocytes (Figure 4B(b), 4B(c)). This transplanted tissue accounted for 11.0 $\pm$ 1.6% of the scar area, representing a substantial size. Additionally, transplanted cardiomyocytes were identified using human-specific cardiac troponin I antibodies, and no signs of graft rejection were observed in the hearts of four out of five animals that received the transplant.

Furthermore, the cardiomyocytes of the transplanted animals exhibited a similar myogenic pattern to those of the host cardiomyocytes, with equivalent cTnT expression and sarcomere length (Figure 2B), indicating that the transplanted myocardial tissue had matured to nearly the same level as the host cardiomyocytes by 12 weeks post-transplantation.

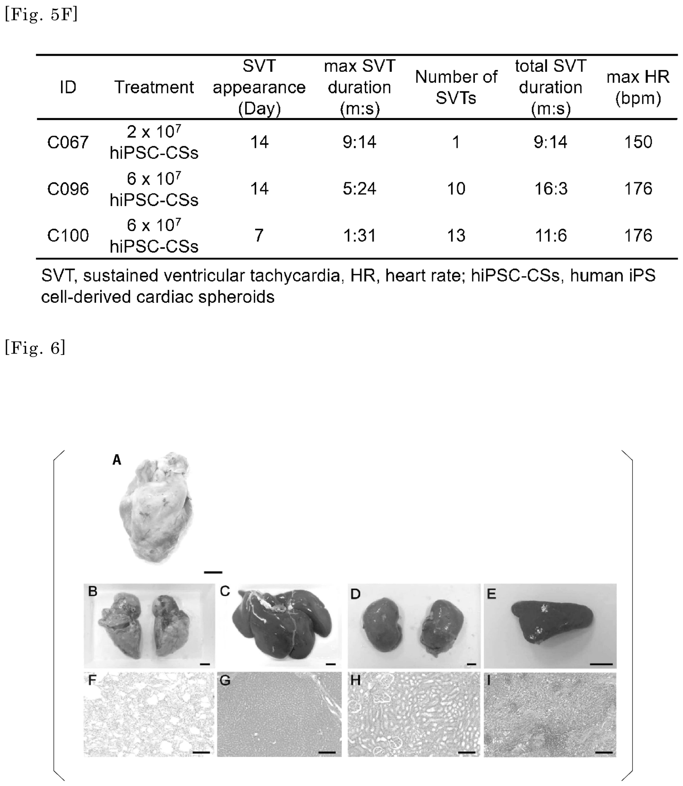

In Example 3(3), no signs of ectopic cardiomyocyte transplantation were observed in the lungs, liver, kidneys, or spleen of animals that received transplantation of human iPS cell-derived cardiac spheroids (equivalent to $6 \times 10^7$ cardiomyocytes) (Figures 6A-I).

All transplanted cells were cTnT-positive cardiomyocytes. Most of the cardiomyocytes were MLC2v-positive mature ventricular subtypes, but only a small number of cardiomyocytes expressed MLC2a (Figure 4A(F)).Ki67-positive cells were also present in the transplanted area, but most were cTnT-negative non-cardiac cells, and Ki67-positive CMs were extremely rare at 12 weeks post-transplantation (Figure 4A(G)).The transplanted cardiomyocytes were well vascularized (Figure 4A(J), 4A(M)) and abundantly expressed connexin 43 and cadherin (Figure 4A(H), (I), (K), (L)).These results indicated that the transplanted cardiomyocytes were surviving at a maturity level similar to that of host cardiomyocytes.

**(Echocardiography)**

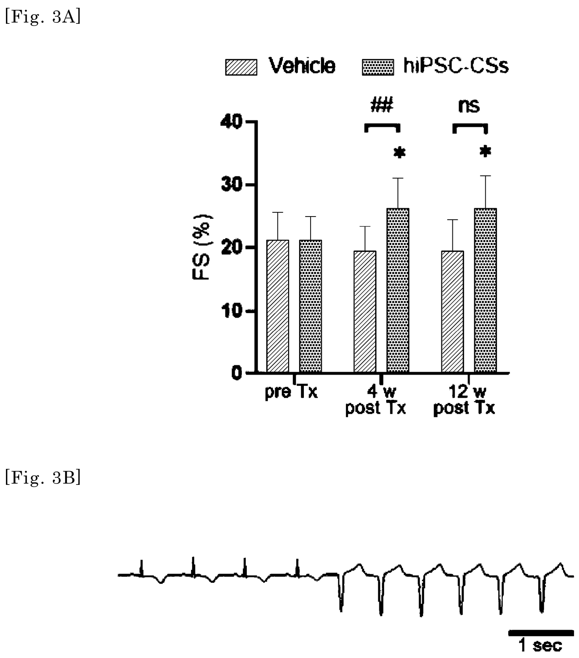

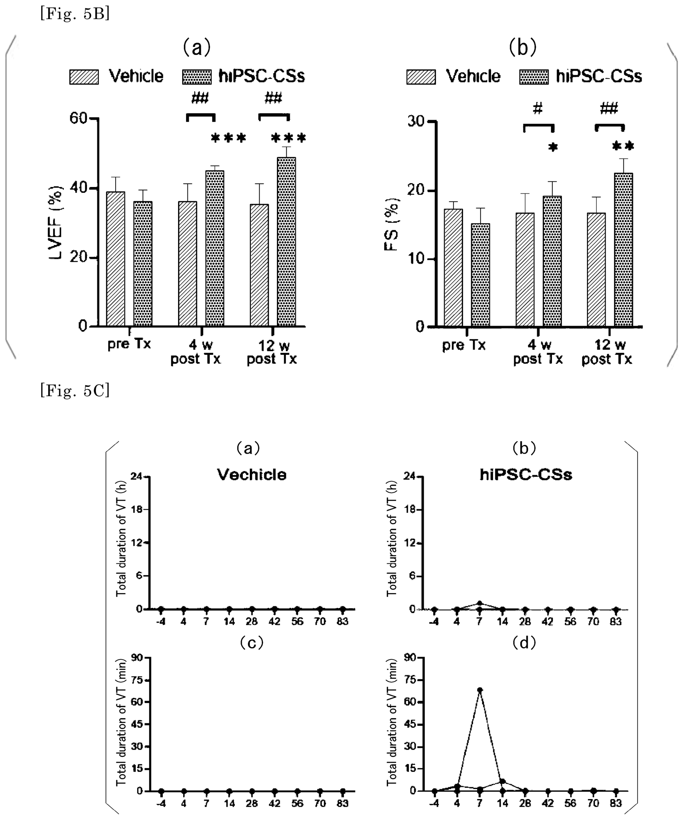

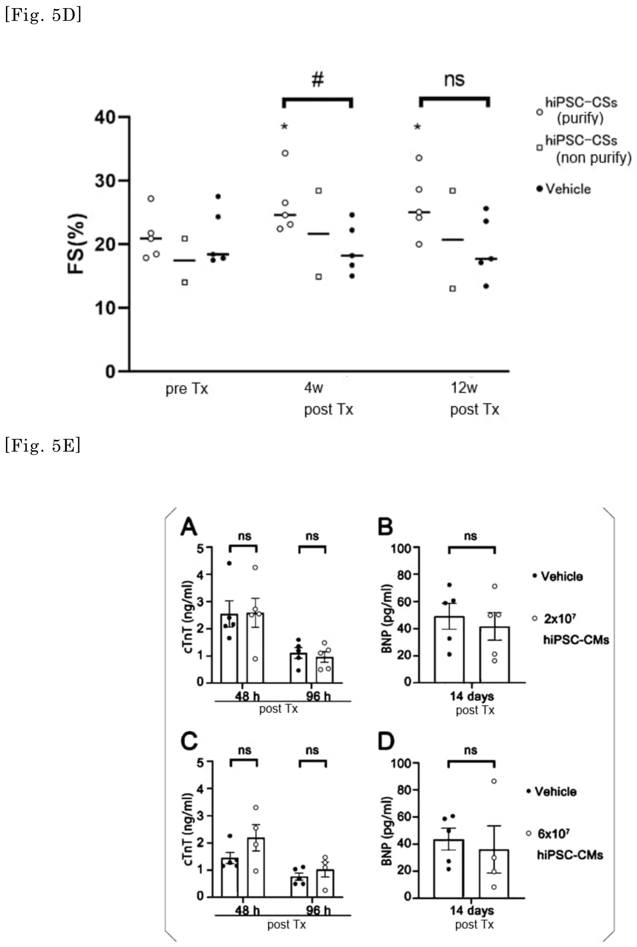

In Example 3(3), echocardiographic analysis was performed to measure the left ventricular short-axis diameter (FS) in animals and vehicles that received transplantation of human iPS cell-derived cardiac spheroids (equivalent to $2 \times 10^7$ cardiomyocytes) at pre-transplantation, 4 weeks post-transplantation, and 12 weeks post-transplantation. The results are shown in Figure 3A. As clearly shown in Figure 3A, animals that received cardiac spheroids transplantation exhibited a significant improvement in contractile function at 4 weeks post-transplantation. Additionally, echocardiograms of animals that received cardiac spheroids transplantation equivalent to $6 \times 10^7$ cardiomyocytes were performed before transplantation, 4 weeks post-transplantation, and 12 weeks post-transplantation, and the LVEF measurement values are shown in Figure 5A and Figure 5B, respectively. Echocardiograms showed that animals receiving cardiac spheroids transplantation exhibited higher fractional shortening than those receiving vehicle transplantation, and this advantage persisted up to 12 weeks post-transplantation. On the other hand, we also performed an experiment using human iPS cell-derived cardiomyocytes that underwent only cardiomyocyte differentiation and not purification, and transplanted them into cynomolgus monkeys using the same method as in Example 3(3).For both the spheroids produced from an unpurified population of cardiomyocytes transplanted animals and the vehicle-transplanted animals, left ventricular short-axis diameter (FS) was measured by echocardiography before transplantation, 4 weeks post-transplantation, and 12 weeks post-transplantation. The results were compared with those obtained from animals transplanted with cardiac spheroids, as shown in Figure 5D. As shown in Figure 5D, the FS values in the animals transplanted with iPSC-derived cardiac spheroids that were produced from an unpurified population of cardiomyocyte were significantly lower than those in the vehicle-transplanted animals. As clearly shown in Figure 5D, animals that received cardiac spheroids produced from a purified population of cardiomyocytes differentiated from human iPS cells (hiPSC-CSCs (purified)) exhibited significantly higher FS values compared to animals that received cardiac spheroids produced from an unpurified population of cardiomyocytes differentiated from human iPS cells (hiPSC-CSCs (unpurified)). Animals that received cardiac spheroids produced from hiPSC-CSCs (purified) showed significantly improved contractile function at four weeks post-transplantation compared to animals that received cardiac spheroids produced from hiPSC-CSCs (unpurified).

**(Computed Tomography)**

In Example 3, animals that received a transplant of human iPS cell-derived cardiac spheroids (equivalent to $6 \times 10^7$ cardiomyocytes) and vehicle-transplanted animals underwent computed tomography (CT) on the day before transplantation, 28 days after transplantation, and 84 days after transplantation. The left ventricular ejection fraction (LVEF) calculated from the results is shown in Figure 5B(a). At 4 weeks and 12 weeks post-transplantation, the LVEF in the animals that received transplant of cardiac spheroids produced from hiPSC-CSCs was significantly higher than that in the vehicle-transplanted animals.

**(Blood tests)**

Peripheral blood was collected 2 days and 4 days post-transplantation from the animals that received transplant of human iPS cell-derived cardiac spheroids ($2 \times 10^7$ cardiac myocytes equivalent (Fig. 5E-A and B),$6 \times 10^7$ cardiomyocytes (Figure 5E-C and D) in Example 3 (3), and cardiac troponin T (cTnT) levels were measured. Additionally, peripheral blood was collected 14 days post-transplantation, and serum levels of brain natriuretic peptide (BNP) were measured. These results are shown in Figure E. As shown in Figures 5E-A and C, both animals that received transplant of human iPS cell-derived cardiac spheroids and vehicle-transplanted animals exhibited elevated serum cTnT levels, confirming an ischemic state (Figures 5E-A and C). Additionally, serum BNP levels were found to be equivalent in both vehicle-transplanted animals and animals that received transplant of human iPS cell-derived cardiac spheroids.

**(Holter Electrocardiogram)**

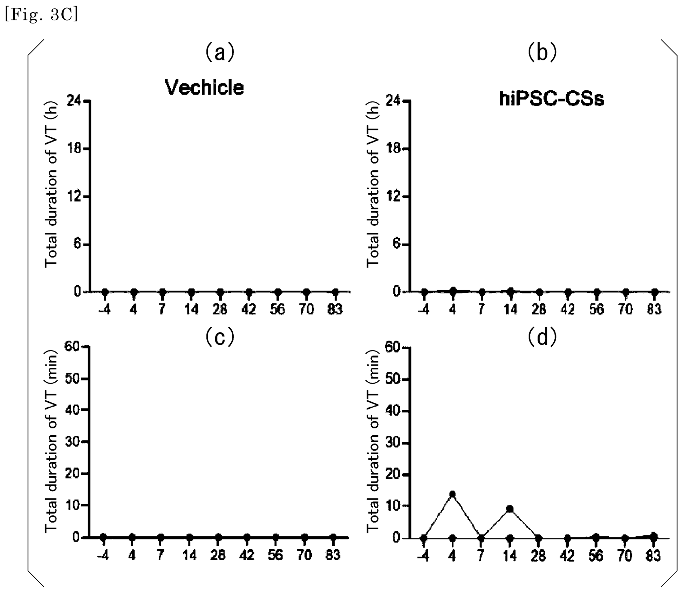

In animals transplanted with cardiac spheroids produced from a population of cardiomyocytes differentiated from human iPS cells, and in vehicle-transplanted animals, electrocardiograms were recorded by Holter electrocardiography before transplantation, on days 4, 7, and 14 after transplantation, and every two weeks thereafter. No significant ventricular arrhythmias were observed in the vehicle recipients (Figure 3C(a) and 3C(c)). Additionally, in four out of five animals transplanted with cardiac spheroids produced from a population of cardiomyocytes differentiated from human iPS cells, no or only minimal ventricular arrhythmias were observed during the experimental period (Figure 3C(b) and 3C(d)). Notably, one animal (ID: C067 in Figure 5D) that received transplantation exhibited transient ventricular tachycardia (9 minutes and 14 seconds within a single day) up to two weeks post-transplantation (Figure 5D), but no ventricular arrhythmias were observed thereafter (Figures 3B, 3C(b), and 3C(d)).

Additionally, the same analysis was performed on animals transplanted with cardiac spheroids produced from a population of cardiomyocytes differentiated from human iPS cells (equivalent to $6 \times 10^7$ cardiomyocytes) and on vehicle-transplanted animals in Example 3. The results are shown in Figure 5C. As evident from Figure 5C, in animals transplanted with cardiac spheroids produced from a population of cardiomyocytes differentiated from human iPS cells, two out of four animals (ID: C096 and C100 in Figure 5) exhibited sustained ventricular arrhythmias, but no sustained ventricular arrhythmias were observed after day 14 (Figure 5C(b) and 5C(d)). These ventricular arrhythmias had a maximum heart rate of 176 bpm and lasted only 11 minutes and 6 seconds and 16 minutes and 3 seconds, respectively (Figure 5D).

The transient ventricular arrhythmias observed in some animals transplanted with cardiac spheroids produced from a population of cardiomyocytes differentiated from human iPS cells in Example 3 were characterized by a very short duration and low frequency compared to previous studies (Shiba Y. et al., Nature. 2016; 538:388-391). The short duration and low frequency of these arrhythmias may be explained by differences in the transplanted human iPS cell-derived cardiomyocytes. Such differences are considered to result from factors including the high purity and maturity of the cardiomyocytes in the population produced in Example 1, and the high ratio of viable cells in the cardiomyocyte population transplanted into *Cynomolgus monkeys* in Example3.

The inventors have been developing a method of directly transplanting human pluripotent stem cell-derived cardiomyocytes (PSC-CMs) into the myocardial layer, as performed in Example 3. This method carries potential risks associated with needle injection, such as tissue damage, bleeding, thrombosis, and cardiac edema; however, the transplanted PSC-CMs were shown to contract in synchrony with adjacent recipient cardiomyocytes, a phenomenon referred to as cardiac regeneration therapy. The cardiac spheroids produced in Example 2 have several advantages and may serve as an ideal active component for cardiac regeneration therapy. First, the preparation of cardiac spheroids is straightforward, involving the seeding of a population of cardiomyocytes onto a specific culture plate and allowing them to naturally form cellular aggregates. This simplicity is particularly advantageous when preparing large quantities of cardiomyocyte populations, such as those required for human cardiac regeneration therapy. Second, cardiac spheroids maintain a survival rate of at least 24 hours in normal physiological saline at 4°C, enabling the pharmaceutical composition to be transported from the CPC to multiple medical institutions without the need for cell processing prior to transplantation. Third, the cardiac spheroids can be easily expanded in vitro, enabling the production of a large number of cells for transplantation.

Finally, the cardiac spheroids manufactured in Example 2 effectively engrafted into recipient cardiac muscle tissue without the use of additives. Animals transplanted with cardiac spheroids containing $6 \times 10^7$ cardiomyocytes, as performed in Example 3, exhibited significant functional recovery of contraction and a substantial cardiomyocyte graft occupying $11.0 \pm 1.6\%$ of the scar area even 12 weeks post-transplantation.

The post-transplantation arrhythmias observed in Example 3 exhibited similar characteristics to those reported in previous studies (Shiba Y. et al., Nature 2016; 538:388-391), in which the purity of cardiomyocytes was 63%. However, in Example 3, the duration of arrhythmias was significantly shorter and the incidence rate was lower compared to previous studies (Figure 5F). The lower incidence of arrhythmias after transplantation may be explained by differences in the origin of the transplanted cardiomyocytes. In previous studies, the cardiomyocytes were derived from homologous monkeys, whereas in Example 3, they were derived from allogeneic human iPS cells.

Nevertheless, other allogeneic transplantation studies (Liu Y.W. et al., Nat Biotechnol. 2018; 36:597-605; Chong J.J. et al., Nature 2014; 510:273-277; Romagnuolo R. et al., Stem Cell Reports 2019; 12:967-981; Wahiba D. et al., Circulation 2022; 145:1412-1426 [US20200407687]) reported a high incidence of persistent ventricular arrhythmias post-transplantation. In other xenotransplantation studies, such as Liu Y.W. et al. (Nat Biotechnol. 2018; 36:597-605), the purity of transplanted cardiomyocytes was 86-99%, but the frequency and onset time of persistent ventricular arrhythmias were high. Additionally, in Chong J.J. et al. (Nature 2014; 510:273-277), the purity of transplanted cardiomyocytes was $73 \pm 12\%$, yet the frequency and onset time of persistent ventricular arrhythmias were also high. Furthermore, in Romagnuolo R. et al. (Stem Cell Reports 2019; 12:967-981), the purity of transplanted cardiomyocytes was $81.9 \pm 3.9\%$, with a viable cell ratio of $80.7 \pm 2.4\%$, but the frequency and duration of ventricular arrhythmias remained high. Wahiba D. et al. (Circulation 2022; 145:1412-1426) reported that the spontaneous beating rate of transplanted cardiomyocytes was 45 bpm on day 20 of culture and approximately 30 bpm on day 40, yet ventricular arrhythmias were still observed.

From these findings, it was first demonstrated that at least two conditions must be satisfied to achieve a low incidence and short onset time of ventricular arrhythmias after transplantation, as observed in Example 3 (1) the purity of cardiomyocytes in the transplanted cardiac spheroids was 99.8%, and (2) the spontaneous beating rate was $34 \pm 13$ beats per minute. Additionally, the human iPS cell-derived cardiomyocytes manufactured in Example 1 underwent a differentiation and purification process totaling a maximum of 25 days, differing from the long-term culture methods used to produce matured cardiomyocytes.

In summary, when clinical-grade iPSC-CM preparations were transplanted into infarcted primate hearts, transient non-fatal arrhythmias occurred, but long-term graft survival and improvement in contractile function were confirmed. These arrhythmias were considered manageable within acceptable safety limits. The human iPS cell-derived cardiomyocyte populations produced in Example 1 exhibited the following characteristics: over 90% of the cells were cardiac troponin T-positive (Figure 1A(b)), and spontaneous contractions occurred at a rate of 0-60 beats per minute (Figure 1C).Additionally, the cardiac spheroids produced in Example 2 were manufactured using cardiomyocytes derived from Example 1 within 30 days of the initiation of differentiation (excluding the freezing storage period). These cardiomyocyte populations are expected to regenerate damaged areas of the recipient heart by electrically synchronizing with recipient cardiomyocytes, maintaining their maturity, and surviving long-term (Figures 2B, 3A, 4A, 4B, 5A, 5B).Furthermore, among the animals transplanted with these cardiac spheroids, some exhibited no ventricular arrhythmias, and the arrhythmias observed in others were significantly less frequent than in previous studies, transient, and clinically manageable (Figures 3C, 5C, 5D).

Based on these properties, the cardiomyocytes produced in Example 1 and the cardiac spheroids manufactured in Example 2 can be used as cell therapy products suitable for clinical application.

**INDUSTRIAL APPLICABILITY**

According to the present invention, there are provided:

a human pluripotent stem cell-derived cardiomyocyte population and cardiac spheroid that may satisfy the conditions necessary for clinical application;
a pharmaceutical composition comprising the aforementioned cardiomyocyte population and/or cardiac spheroid as an active ingredient;
a method for manufacturing the aforementioned cardiomyocyte population; and
a method for manufacturing the aforementioned cardiac spheroid.

The foregoing description illustrates preferred embodiments of the present invention; however, the invention is not limited thereto.

Various additions, omissions, substitutions, and modifications can be made without departing from the spirit and scope of the present invention.

The present invention is not to be construed as being limited by the above description, but only by the scope of the appended claims.

**Claims**

1. A population of cardiomyocytes comprising ventricular cardiomyocytes differentiated from human pluripotent stem cells,
   wherein the population of cardiomyocytes has the following characteristics:

   (1) cells positive for cardiac troponin T constitute 90% or more of all viable cells,
   (2) the spontaneous beating rate is 0 to 60 beats per minute, and
   (3) the population of cardiomyocyte is within 30 days from initiation of differentiation.

2. The population of cardiomyocytes according to claim 1, wherein the spontaneous beating rate is 0 to 50 beats per minute.

3. A pharmaceutical composition comprising at least one selected from the group consisting of the population of cardiomyocytes according to claim 1 or 2, and cardiac spheroids obtained by spheroidizing the population of cardiomyocytes, and a pharmaceutically acceptable carrier.

4. The pharmaceutical composition according to claim 3, wherein the proportion of viable cells in the population of cardiomyocytes is 80% or more of all cells.

5. The pharmaceutical composition according to claim 3, wherein cells constituting the cardiac spheroids account for 60% or more of all cells contained in the pharmaceutical composition.

6. The pharmaceutical composition according to claim 3, for use in the treatment of heart failure.

7. A method for producing the population of cardiomyocytes according to claim 1 or 2, comprising:

   (a) expanding human pluripotent stem cells in culture;
   (b) culturing the expanded human pluripotent stem cells under conditions for differentiation into cardiomyocytes to produce a cell population comprising 60% or more cardiomyocytes; and

(c) removing human pluripotent stem cells and non-cardiomyocyte cells from the cell population.

8. A cardiac spheroid obtained by spheroidizing the population of cardiomyocytes according to claim 1 or 2.

9. The cardiac spheroid according to claim 8, wherein the diameter of the spheroid is 50 to 300 $\mu$m.

10. A method for producing a cardiac spheroid, comprising suspending the population of cardiomyocytes according to claim 1 or 2 in a medium and subsequently culturing the suspension in a culture vessel having microwells at the bottom.

[Fig. 1A]

(a)          (b)

81.0%          99.8%

Cardiac troponin T

[Fig.1B]

[Fig. 1C]

[Fig. 1D]

[Fig. 1E]

[Fig. 1F]

[Fig.1G]

[Fig. 2A]

[Fig.2B]

sarcomere length

[Fig. 2C]

[Fig. 2D]

[Fig. 2E]

[Fig. 2F]

[Fig. 2G]

[Fig. 3A]

[Fig. 3B]

[Fig. 3C]

[Fig. 4A]

[Fig. 4B]

(a)

**Scar area**

(b)

**Grft area**

(c)

**Graft area**

[Fig. 5A]

[Fig. 5B]

[Fig. 5C]

[Fig. 5D]

[Fig. 5E]

[Fig. 5F]

| ID | Treatment | SVT appearance (Day) | max SVT duration (m:s) | Number of SVTs | total SVT duration (m:s) | max HR (bpm) |
|---|---|---|---|---|---|---|
| C067 | $2 \times 10^7$ hiPSC-CSs | 14 | 9:14 | 1 | 9:14 | 150 |
| C096 | $6 \times 10^7$ hiPSC-CSs | 14 | 5:24 | 10 | 16:3 | 176 |
| C100 | $6 \times 10^7$ hiPSC-CSs | 7 | 1:31 | 13 | 11:6 | 176 |

SVT, sustained ventricular tachycardia, HR, heart rate; hiPSC-CSs, human iPS cell-derived cardiac spheroids

[Fig. 6]

## INTERNATIONAL SEARCH REPORT

| International application No. |
|---|
| **PCT/JP2024/019949** |

| | |
|---|---|
| **A. CLASSIFICATION OF SUBJECT MATTER** | |

*C12N 5/077*(2010.01)i; *A61K 35/34*(2015.01)i; *A61P 9/04*(2006.01)i; *C12N 5/0789*(2010.01)i; *C12N 5/10*(2006.01)i
FI: C12N5/077; C12N5/0789; C12N5/10; A61K35/34; A61P9/04

According to International Patent Classification (IPC) or to both national classification and IPC

**B. FIELDS SEARCHED**

Minimum documentation searched (classification system followed by classification symbols)

C12N5/077; A61K35/34; A61P9/04; C12N5/0789; C12N5/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN); PubMed; Google

**C. DOCUMENTS CONSIDERED TO BE RELEVANT**

| Category* | Citation of document, with indication, where appropriate, of the relevant passages | Relevant to claim No. |
|---|---|---|
| X | MA, J. et al., High purity human-induced pluripotent stem cell-derived cardiomyocytes: electrophysiological properties of action potentials and ionic currents., Am. J. Physiol. Heart Circ. Physiol., 2011, vol. 301, pp. H2006-H2017<br>    p. H2007, left column, first paragraph, p. H2008, fig. 1, fig. 2 (Venticular-like) | 1-2, 7 |
| Y | | 1-10 |
| X | SCHMID, C. et al., Ion channel expression and electrophysiology of singular human (primary and induced pluripotent stem cell-derived) cardiomyocytes., Cells, 2021, vol. 10, no. 3370, pp. 1-21<br>    Materials and Methods, table 2 (Ventr. PCs), table 3 (TNNT2) | 1-2, 7 |
| Y | | 1-10 |
| Y | 柴祐司, 102. 霊長類iPS細胞を用いた心筋再生治療実用化研究., 上原記念生命科学財団研究報告集, 2019, vol. 33, pp. 1-4, non-official translation (SHIBA, Yuji. 102. Study of practical use of cardiac muscle regeneration treatment using primate iPS cells. Research Reports of the Uehara Memorial Foundation.)<br>    text, drawings | 1-10 |

☑ Further documents are listed in the continuation of Box C.     ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 August 2024** | **20 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

| International application No. |
| --- |
| **PCT/JP2024/019949** |

### C. DOCUMENTS CONSIDERED TO BE RELEVANT

| Category* | Citation of document, with indication, where appropriate, of the relevant passages | Relevant to claim No. |
| --- | --- | --- |
| A | 市村創 外, 多能性幹細胞由来心筋細胞移植と移植後不整脈の現状., 血管, 2022, vol. 45, no. 3, pp. 17-23, (Japanese Journal of Circulation Research), non-official translation (ICHIMURA, Hajime et al. Current conditions of pluripotent stem cell-derived cardiomyocyte transplantation and post-transplantation arrhythmia.)<br>text, drawings | 1-10 |
| A | WO 2016/010165 A1 (KEIO UNIVERSITY) 21 January 2016 (2016-01-21)<br>claims, examples | 1-10 |
| A | WO 2018/074457 A1 (KEIO UNIVERSITY) 26 April 2018 (2018-04-26)<br>claims, examples | 1-10 |

Form PCT/ISA/210 (second sheet) (July 2022)

## INTERNATIONAL SEARCH REPORT
### Information on patent family members

International application No.

**PCT/JP2024/019949**

| Patent document cited in search report | | | Publication date (day/month/year) | Patent family member(s) | | | Publication date (day/month/year) |
|---|---|---|---|---|---|---|---|
| WO | 2016/010165 | A1 | 21 January 2016 | US claims, examples EP | 2017/0327787 3170894 | A1 A1 | |
| WO | 2018/074457 | A1 | 26 April 2018 | US claims, examples EP | 2019/0300857 3527657 | A1 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2023088954 A
- WO 2007088874 A
- WO 2016010165 A
- WO 2018074457 A
- US 20200407687

- WO 2018181342 A
- WO 20183031074457 A
- WO 2009017524 A
- WO 2020013125 A
- US 20200407687 A

### Non-patent literature cited in the description

- **LAFLAMME MA. et al.** Cardiomyocytes derived from human embryonic stem cells in pro-survival factors enhance function of infarcted rat hearts. *Na Biotechnol.*, 2007, vol. 25, 1015-1024
- **FUNAKOSHI S. et al.** Enhanced engraftment, proliferation and therapeutic potential in heart using optimized human iPSC-derived cardiomyocytes.. *Sci Rep.*, 2016, vol. 6, 19111
- **SHIBA Y. et al.** Human ES-cell-derived cardiomyocytes electrically couple and suppress arrhythmias in injured hearts. *Nature*, 2012, vol. 489, 322-5
- **DHAHRI W. et al.** Vitro Matured Human Pluripotent Stem Cell-Derived Cardiomyocytes Form Grafts With Enhanced Structure and Function in Injured Hearts. *Circulation*, 2022, vol. 145, 1412-1426
- **LIW YW. et al.** Human embryonic stem cell-derived cardiomyocytes restore function in infarcted hearts of non-human primates. *Nat Biotechnol.*, 2018, vol. 36, 597-605
- **CHONG JJ. et al.** Human embryonic-stem-cell-derived cardiomyocytes regenerate non-human primate hearts. *Nature*, 2014, vol. 510 (2), 73-7
- **SHIBA Y. et al.** Allogeneic transplantation of iPS cell-derived cardiomyocytes regenerates primate hearts. *Nature*, 2016, vol. 538, 388-391
- **ROMAGNUOLO R. et al.** Human Embryonic Stem Cell-Derived Cardiomyocytes Regenerate the Infarcted Pig Heart but Induce Ventricular Tachyarrhythmias. *Stem Cell Reports.*, 2019, vol. 12, 967-981
- **NAKAMURA K. et al.** Pharmacologic therapy for engraftment arrhythmia induced by transplantation of human cardiomyocytes. *Stem Cell Reports*, 2021, vol. 16, 2473-2487
- **MARCHIANO S. et al.** Gene editing to prevent ventricular arrhythmias associated with cardiomyocyte cell therapy. *Cell Stem Cell*, 2023, vol. 30, 396-414
- **DHAHRI W. et al.** *Circulation*, 2022, vol. 145, 1412-1426

- **THOMSON et al.** *Science*, 1998, vol. 282 (5391), 1145-7
- **LUDWIG et al.** *Nat Methods*, 2006, vol. 3, 637-46
- **KENNEDY et al.** *Blood*, 2007, vol. 109, 2679-87
- *Nat Methods*, 2011, vol. 8, 424-9
- **WANG et al.** *Stem Cell Res.*, 2013, vol. 11 (3), 1103-16
- **NIWA A et al.** *PLoS One.*, 2011, vol. 6 (7), e22261
- **TOHYAMA S. et al.** *Stem Cell Reports*, 2017, vol. 9, 1406-1414
- **TANOSAKI S. et al.** *iScience*, 2020, vol. 23, 101535
- **TANOSAKI S. et al.** *STAR Protocols.*, 2022, vol. 3, 101360
- **TOHYAMA S. et al.** *Cell Metabolism.*, 2016, vol. 23, 663-74
- **TOHYAMA S. et al.** *Cell Stem Cell*, 2013, vol. 12, 127-37
- **KAWAGUCHI S. et al.** *JACC Basic Transl Sci.*, 2021, vol. 6, 239-254
- **TABEI R. et al.** *J Heart Lung Transplant.*, 2019, vol. 38, 203-214
- **TANOSAKI S. et al.** *STAR Protoc*, 2022, vol. 3, 101360
- **TOHYAMA S. et al.** *Cell Metab*, 2016, vol. 23, 663-74
- **ICHIMURA H. et al.** *Sci Rep*, 2020, vol. 10, 11883
- **KAWAGUCHI S. et al.** *JACC Basic Transl Sci*, 2021, vol. 6, 239-254
- **TABEI R. et al.** *J Heart Lung Transplant*, 2019, vol. 38, 203-214
- **ICHIMURA H. et al.** *Sci Rep.*, 2020, vol. 10, 11883
- **KOBAYASHI H. et al.** *Methods Mol Biol.*, 2021, vol. 2320, 295-3023

- **SHIBA Y. et al.** *Nature*, 2016, vol. 538, 388-391
- **LIU Y.W. et al.** *Nat Biotechnol.*, 2018, vol. 36, 597-605
- **CHONG J.J. et al.** *Nature*, 2014, vol. 510, 273-277
- **ROMAGNUOLO R. et al.** *Stem Cell Reports*, 2019, vol. 12, 967-981
- **WAHIBA D. et al.** *Circulation*, 2022, vol. 145, 1412-1426